(19) Europäisches Patentamt / European Patent Office / Office européen des brevets

(11) **EP 1 611 077 B1**

(12) **EUROPÄISCHE PATENTSCHRIFT**

(45) Veröffentlichungstag und Bekanntmachung des Hinweises auf die Patenterteilung:
**19.03.2008 Patentblatt 2008/12**

(21) Anmeldenummer: **04722160.1**

(22) Anmeldetag: **20.03.2004**

(51) Int Cl.:
**C07C 57/05** *(2006.01)*

(86) Internationale Anmeldenummer:
**PCT/EP2004/002936**

(87) Internationale Veröffentlichungsnummer:
**WO 2004/085369 (07.10.2004 Gazette 2004/41)**

(54) **VERFAHREN DER HETEROGEN KATALYSIERTEN PARTIELLEN GASPHASENOXIDATION VON PROPEN ZU ACRYLSÄURE**

METHOD FOR THE HETEROGENEOUSLY CATALYSED PARTIAL GAS PHASE OXIDATION OF PROPENE TO FORM ACRYLIC ACID

PROCEDE D'OXYDATION PARTIELLE EN PHASE GAZEUSE DE PROPENE EN ACIDE ACRYLIQUE PAR CATALYSE HETEROGENE

(84) Benannte Vertragsstaaten:
**AT BE BG CH CY CZ DE DK EE ES FI FR GB GR HU IE IT LI LU MC NL PL PT RO SE SI SK TR**

(30) Priorität: **25.03.2003 DE 10313213**
**05.06.2003 US 475790 P**

(43) Veröffentlichungstag der Anmeldung:
**04.01.2006 Patentblatt 2006/01**

(73) Patentinhaber: **BASF SE**
**67056 Ludwigshafen (DE)**

(72) Erfinder:
• **PETZOLDT, Jochen**
**68163 Mannheim (DE)**
• **DIETERLE, Martin**
**68167 Mannheim (DE)**
• **ARNOLD, Heiko**
**Zijin Villas, Nanjing 210014 (CN)**
• **RUPPEL, Wilhelm**
**68163 Mannheim (DE)**
• **MÜLLER-ENGEL, Klaus-Joachim**
**76297 Stutensee (DE)**

(56) Entgegenhaltungen:
**DE-A- 3 042 468        DE-A- 19 955 168**

**Beschreibung**

Vorliegende Erfindung betrifft ein Verfahren der heterogen katalysierten partiellen Gasphasenoxidation von Propen zu Acrylsäure, bei dem man ein Propen, molekularen Sauerstoff und wenigstens ein Inertgas enthaltendes Reaktionsgasausgangsgemisch 1, das den molekularen Sauerstoff und das Propen in einem molaren Verhältnis $O_2$: $C_3H_6 \geq 1$ enthält, in einer ersten Reaktionsstufe mit der Maßgabe über eine Festbettkatalysatorschüttung 1, deren Aktivmasse wenigstens ein die Elemente Mo, Fe und Bi enthaltendes Multimetalloxid ist, führt, dass

- die Festbettkatalysatorschüttung 1 in zwei räumlich aufeinanderfolgenden Temperaturzonen A, B angeordnet ist,
- sowohl die Temperatur der Temperaturzone A als auch die Temperatur der Temperaturzone B eine Temperatur im Bereich von 290 bis 380°C ist,
- die Festbettkatalysatorschüttung 1 aus wenigstens zwei räumlich aufeinander folgenden Festbettkatalysatorschüttungszonen besteht, wobei die volumenspezifische Aktivität innerhalb einer Festbettkatalysatorschüttungszone im wesentlichen konstant ist und in Strömungsrichtung des Reaktionsgasgemisches 1 beim Übergang von einer Festbettkatalysatorschüttungszone in eine andere Festbettkatalysatorschüttungszone sprunghaft zunimmt,
- sich die Temperaturzone A bis zu einem Umsatz des Propens von 40 bis 80 mol-% erstreckt,
- bei einmaligem Durchgang des Reaktionsgasausgangsgemisches 1 durch die gesamte Festbettkatalysatorschüttung 1 der Propenumsatz $\geq 90$ mol-% und die Selektivität der Acroleinbildung sowie der Acrylsäurenebenproduktbildung zusammengenommen und bezogen auf umgesetztes Propen $\geq 90$ mol-% beträgt,
- die zeitliche Abfolge, in der das Reaktionsgasgemisch 1 die Temperaturzonen A, B durchströmt, der alphabetischen Abfolge der Temperaturzonen A, B entspricht,
- die Belastung der Festbettkatalysatorschüttung 1 mit dem im Reaktionsgasausgangsgemisch 1 enthaltenen Propen $\geq 70$ Nl Propen/l Festbettkatalysatorschüttung 1 h beträgt, und
- die Differenz $T^{maxA}$ - $T^{maxB}$, gebildet aus der höchsten Temperatur $T^{maxA}$, die das Reaktionsgasgemisch 1 innerhalb der Temperaturzone A aufweist, und der höchsten Temperatur $T^{maxB}$, die das Reaktionsgasgemisch 1 innerhalb der Temperaturzone B aufweist, $\geq 0$°C beträgt,

die Temperatur des die erste Reaktionsstufe verlassenden Produktgasgemisches durch Kühlung gegebenenfalls verringert und dem Produktgasgemisch gegebenenfalls molekularen Sauerstoff und/oder Inertgas zugibt, und danach das Produktgasgemisch als Acrolein, molekularen Sauerstoff und wenigstens ein Inertgas enthaltendes Reaktionsgasausgangsgemisch 2, das den molekularen Sauerstoff und das Acrolein in einem molaren Verhältnis $O_2$:$C_3H_4O \geq 0,5$ enthält, in einer zweiten Reaktionsstufe mit der Maßgabe über eine Festbettkatalysatorschüttung 2, deren Aktivmasse wenigstens ein die Elemente Mo und V enthaltendes Multimetalloxid ist, führt, dass

- die Festbettkatalysatorschüttung 2 in zwei räumlich aufeinanderfolgenden Temperaturzonen C, D angeordnet ist,
- sowohl die Temperatur der Temperaturzone C als auch die Temperatur der Temperaturzone D eine Temperatur im Bereich von 230 bis 320°C ist,
- die Festbettkatalysatorschüttung 2 aus wenigstens zwei räumlich aufeinander folgenden Festbettkatalysatorschüttungszonen besteht, wobei die volumenspezifische Aktivität innerhalb einer Festbettkatalysatorschüttungszone im wesentlichen konstant ist und in Strömungsrichtung des Reaktionsgasgemisches 2 beim Übergang von einer Festbettkatalysatorschüttungszone in eine andere Festbettkatalysatorschüttungszone sprunghaft zunimmt,
- sich die Temperaturzone C bis zu einem Umsatz des Acroleins von 45 bis 85 mol-% erstreckt,
- bei einmaligem Durchgang des Reaktionsgasausgangsgemisches 2 durch die gesamte Festbettkatalysatorschüttung der Acroleinumsatz $\geq 90$ mol-% und die Selektivität der Acrylsäurebildung, bezogen auf über beide Reaktionsstufen umgesetztes Propen, $\geq 80$ mol-% beträgt,
- die zeitliche Abfolge, in der das Reaktionsgasgemisch 2 die Temperaturzonen C, D durchströmt, der alphabetischen Abfolge der Temperaturzonen C, D entspricht,
- die Belastung der Festbettkatalysatorschüttung 2 mit dem im Reaktionsgasausgangsgemisch 2 enthaltenen Acrolein $\geq 90$ Nl Acrolein/l Festbettkatalysatorschüttung 2 h beträgt, und
- die Differenz $T^{maxC}$ - $T^{maxD}$, gebildet aus der höchsten Temperatur $T^{maxC}$, die das Reaktionsgasgemisch innerhalb der Temperaturzone C aufweist, und die höchsten Temperatur $T^{maxD}$, die das Reaktionsgasgemisch innerhalb der Temperaturzone D aufweist, $\geq 0$°C beträgt.

Acrylsäure ist ein bedeutendes Monomeres, das als solches oder in Form seiner Acrylester zur Erzeugung von z.B. als Klebstoffen geeigneten Polymerisaten Verwendung findet.

Das vorgenannte Verfahren der zweistufigen heterogen katalysierten partiellen Gasphasenoxidation von Propen zu Acrylsäure ist z.B. aus der DE-A 19927624, aus der DE-A 19948523, aus der WO 00/53557, aus der DE-A 19948248 und aus der WO 00/53558 sowie DE-A-30 42 468 prinzipiell bekannt.

Neben molekularem Sauerstoff und den Reaktanden enthalten die Reaktionsgasausgangsgemische 1 und 2 u.a. deshalb Inertgas, um das Reaktionsgas außerhalb des Explosionsbereichs zu halten.

Eine Zielsetzung einer solchen zweistufigen heterogen Katalysierten partiellen Gasphasenoxidation von Propen zu Acrylsäure besteht darin, bei einmaligem Durchgang des Reaktionsgasgemisches durch die beiden Reaktionsstufen unter ansonsten vorgegebenen Randbedingungen eine möglichst hohe Selektivität $S^{AA}$ an Acrylsäure zu erzielen (das ist die Molzahl von zu Acrylsäure umgesetztem Propen, bezogen auf die Molzahl an umgesetztem Propen).

Eine weitere Zielsetzung besteht darin, bei einmaligem Durchgang des Reaktionsgasgemisches durch die beiden Reaktionsstufen unter ansonsten vorgegebenen Randbedingungen einen möglichst hohen Umsatz $U^P$ an Propylen zu erzielen (das ist die Molzahl von umgesetztem Propen, bezogen auf die Molzahl an eingesetztem Propen).

Ist bei hohem $U^P$ die $S^{AA}$ hoch, entspricht dies einer hohen Ausbeute $A^{AA}$ an Acrylsäure ($A^{AA}$ ist das Produkt $U^P \cdot S^{AA}$; d.h., die Molzahl von zu Acrylsäure umgesetztem Propen, bezogen auf die Molzahl an eingesetztem Propen).

Bei gleich bleibender gegebener Ausbeute $A^{AA}$ ist die Raum-Zeit-Ausbeute um so grö-ßer, je größer die Belastung der Festbettkatalysatorschüttung der ersten Reaktionsstufe (Festbettkatalysatorschüttung 1) mit Propen (darunter wird die Menge an Propen in Normlitem (= Nl; das Volumen in Litern, das die entsprechende Propenmenge bei Normalbedingungen, d.h., bei 25°C und 1 bar, einnehmen würde) verstanden, die als Bestandteil des Reaktionsgasausgangsgemisches 1 pro Stunde durch einen Liter an Festbettkatalysatorschüttung 1 geführt wird) ist.

Die DE-A 19927624, die DE-A 19948523, die WO 00/53557, die DE-A 19948248 und die WO 00/53558 lehren zwar, dass die vorgenannten Ziele mittels des eingangs beschriebenen Verfahrens der zweistufigen heterogen katalysierten partiellen Gasphasenoxidation von Propen zu Acrylsäure prinzipiell zu erzielen sind.

Dies ist insofern von Vorteil, als sich eine geringere volumenspezifische Aktivität innerhalb einer Festbettkatalysatorschüttung bzw. Festbettkatalysatorschüttungszone z.B. dadurch erzielen lässt, dass man die eigentlichen, Aktivmasse tragenden, Katalysatorformkörper mit an Aktivmasse freien inerten Verdünnungskörpern verdünnt, wodurch die Festbettkatalysatorschüttung insgesamt kostengünstiger wird.

Nachteilig an den Lehren der DE-A 19927624, der DE-A 19948523, der WO 00/53557, der DE-A 19948248 und der WO 00/53558 ist jedoch, dass sie kein entsprechendes Ausführungsbeispiel aufweisen und somit die konkrete Ausgestaltung einer solchen Verfahrensweise offen lassen.

In ähnlicher Weise lehrt auch die EP-A 1106598 ein zweistufiges Verfahren der heterogen katalysierten partiellen Gasphasenoxidation von Propen zu Acrylsäure, bei dem die Festbettkatalysatorschüttung der jeweiligen Reaktionsstufe aus mehreren räumlich aufeinander folgenden Festbettkatalysatorschüttungszonen besteht, deren volumenspezifische Aktivität innerhalb einer Festbettkatalysatorsschüttungszone im wesentlichen konstant ist und in Strömungsrichtung des Reaktionsgasgemisches beim Übergang von einer Festbettkatalysatorschüttungszone in eine andere Festbettkatalysatorschüttungszone sprunghaft zunimmt und in mehreren Temperaturzonen angeordnet sein kann. Gemäß der Lehre der EP-A 1106598 ist es dabei zweckmäßig, wenn Festbettkatalysatorschüttungszonen und Temperaturzonen räumlich zusammenfallen.

Eingehende Untersuchungen seitens der Anmelderin haben jedoch ergeben, dass ein räumliches Zusammenfallen von Festbettkatalysatorschüttungszonen und Temperaturzonen für ein optimales Erreichen der verschiedenen angesprochenen Zielsetzungen in der Regel nicht förderlich ist.

Dies ist u.a. darauf zurückzuführen, dass der Übergang von einer Temperaturzone in die andere Temperaturzone und der Übergang von einer Festbettkatalysatorschüttungszone in eine andere Festbettkatalysatorschüttungszone entweder als im wesentlichen gleich wirkende oder als im wesentlichen entgegengesetzt wirkende Maßnahmen ergriffen werden.

Werden sie als im wesentlichen gleich wirkende Maßnahmen ergriffen (z.B. Übergang von einer kälteren Temperaturzone in eine heißere Temperaturzone und Übergang von einer Festbettkatalysatorschüttungszone mit niederer volumenspezifischer Aktivität in eine Festbettkatalysatorschüttungszone mit höherer volumenspezifischer Aktivität; beide Maßnahmen verfolgen den Zweck, die Reaktion zu fördern), so schießt die erzielte Gesamtwirkung im Übergangsbereich häufig über das angestrebte Ziel hinaus und es resultiert z.B. eine Minderung der $S^{AA}$.

Werden sie als im wesentlichen entgegengesetzt wirkende Maßnahmen ergriffen (z.B. Übergang von einer heißeren Temperaturzone in eine kältere Temperaturzone und Übergang von einer Festbettkatalysatorschüttungszone mit niederer volumenspezifischer Aktivität in eine Festbettkatalysatorschüttungszone mit höherer volumenspezifischer Aktivität; die erste Maßnahme verfolgt den Zweck, die Reaktion zu mindern, die zweite Maßnahme verfolgt den Zweck, die Reaktion zu fördern), so neutralisieren sich die Einwirkungen im Übergangsbereich häufig und es resultiert ein geminderter $U^P$.

Die Aufgabe der vorliegenden Erfindung bestand daher darin, ein Verfahren der zweistufigen heterogen katalysierten partiellen Gasphasenoxidation von Propen zu Acrylsäure in einer Mehrzonenanordnung zur Verfügung zu stellen, das die Nachteile der Verfahren des Standes der Technik nicht aufweist.

Demgemäß wurde ein Verfahren der heterogen katalysierten partiellen Gasphasenoxidation von Propen zu Acrylsäure, bei dem man ein Propen, molekularen Sauerstoff und wenigstens ein Inertgas enthaltendes Reaktionsgasausgangsgemisch 1 in einer ersten Reaktionsstufe mit der Maßgabe über eine Festbettkatalysatorschüttung 1, deren Aktivmasse wenigstens ein die Elemente Mo, Fe und Bi enthaltendes Multimetalloxid führt, dass

- die Festbettkatalysatorschüttung 1 in zwei räumlich aufeinanderfolgenden Temperaturzonen A, B angeordnet ist,
- sowohl die Temperatur der Temperaturzone A als auch die Temperatur der Temperaturzone B eine Temperatur im Bereich von 290 bis 380°C ist,
- die Festbettkatalysatorschüttung 1 aus wenigstens zwei räumlich aufeinander folgenden Festbettkatalysatorschüttungszonen besteht, wobei die volumenspezifische Aktivität innerhalb einer Festbettkatalysatorschüttungszone im wesentlichen konstant ist und in Strömungsrichtung des Reaktionsgasgemisches 1 beim Übergang von einer Festbettkatalysatorschüttungszone in eine andere Festbettkatalysatorschüttungszone sprunghaft zunimmt,
- sich die Temperaturzone A bis zu einem Umsatz des Propens von 40 bis 80 mol-% erstreckt,
- bei einmaligem Durchgang des Reaktionsgasausgangsgemisches 1 durch die gesamte Festbettkatalysatorschüttung 1 der Propenumsatz $\geq$ 90 mol-% und die Selektivität der Acroleinbildung sowie der Acrylsäurenebenproduktbildung zusammengenommen und bezogen auf umgesetztes Propen $\geq$ 90 mol-% beträgt,
- die zeitliche Abfolge, in der das Reaktionsgasgemisch 1 die Temperaturzonen A, B durchströmt, der alphabetischen Abfolge der Temperaturzonen A, B entspricht,
- die Belastung der Festbettkatalysatorschüttung 1 mit dem im Reaktionsgasausgangsgemisch 1 enthaltenen Propen $\geq$ 90 Nl Propen/l Festbettkatalysatorschüttung 1 h beträgt, und
- die Differenz $T^{maxA}$ - $T^{maxB}$, gebildet aus der höchsten Temperatur $T^{maxA}$, die das Reaktionsgasgemisch 1 innerhalb der Temperaturzone A aufweist, und der höchsten Temperatur $T^{maxB}$, die das Reaktionsgasgemisch 1 innerhalb der Temperaturzone B aufweist, $\geq$ 0°C beträgt,

die Temperatur des die erste Reaktionsstufe verlassenden Produktgasgemisches durch Kühlung gegebenenfalls verringert und dem Produktgasgemisch gegebenenfalls molekularen Sauerstoff und/oder Inertgas zugibt, und danach das Produktgasgemisch als Acrolein, molekularen Sauerstoff und wenigstens ein Inertgas enthaltendes Reaktionsgasausgangsgemisch 2, das den molekularen Sauerstoff und das Acrolein in einem molaren Verhältnis $O_2:C_3H_4O \geq 0,5$ enthält, in einer zweiten Reaktionsstufe mit der Maßgabe über eine Festbettkatalysatorschüttung 2, deren Aktivmasse wenigstens ein die Elemente Mo und V enthaltendes Multimetalloxid ist, führt, dass

- die Festbettkatalysatorschüttung 2 in zwei räumlich aufeinanderfolgenden Temperaturzonen C, D angeordnet ist,
- sowohl die Temperatur der Temperaturzone C als auch die Temperatur der Temperaturzone D eine Temperatur im Bereich von 230 bis 320°C ist,
- die Festbettkatalysatorschüttung 2 aus wenigstens zwei räumlich aufeinander folgenden Festbettkatalysatorschüttungszonen besteht, wobei die volumenspezifische Aktivität innerhalb einer Festbettkatalysatorschüttungszone im wesentlichen konstant ist und in Strömungsrichtung des Reaktionsgasgemisches 2 beim Übergang von einer Festbettkatalysatorschüttungszone in eine andere Festbettkatalysatorschüttungszone sprunghaft zunimmt,
- sich die Temperaturzone C bis zu einem Umsatz des Acroleins von 45 bis 85 mol-% erstreckt,
- bei einmaligem Durchgang des Reaktionsgasausgangsgemisches 2 durch die gesamte Festbettkatalysatorschüttung 2 der Acroleinumsatz $\geq$ 90 mol-% und die Selektivität der Acrylsäurebildung, bezogen auf über beide Reaktionsstufen umgesetztes Propen, $\geq$ 80 mol-% beträgt,
- die zeitliche Abfolge, in der das Reaktionsgasgemisch 2 die Temperaturzonen C, D durchströmt, der alphabetischen Abfolge der Temperaturzonen C, D entspricht,
- die Belastung der Festbettkatalysatorschüttung 2 mit dem im Reaktionsgasausgangsgemisch 2 enthaltenen Acrolein $\geq$ 70 Nl Acrolein/l Festbettkatalysatorschüttung 2 · h beträgt, und
- die Differenz $T^{maxC}$ - $T^{maxD}$, gebildet aus der höchsten Temperatur $T^{maxC}$, die das Reaktionsgasgemisch innerhalb der Temperaturzone C aufweist, und der höchsten Temperatur $T^{maxD}$, die das Reaktionsgasgemisch innerhalb der Temperaturzone D aufweist, $\geq$ 0°C beträgt,

gefunden, das dadurch gekennzeichnet ist, dass weder der Übergang von der Temperatürzone A in die Temperaturzone B in der Festbettkatalysatorschüttung 1, noch der Übergang von der Temperaturzone C in die Temperaturzone D in der Festbettkatalysatorschüttung 2 (räumlich) mit einem Übergang von einer Festbettkatalysatorschüttungszone in eine andere Festbettkatalysatorschüttungszone zusammenfällt.

Unter der Temperatur einer Temperaturzone wird in dieser Schrift die Temperatur des in der Temperaturzone befindlichen Teils der Festbettkatalysatorschüttung bei Ausübung des erfindungsgemäßen Verfahrens, jedoch in Abwesenheit einer chemischen Reaktion verstanden. Ist diese Temperatur innerhalb der Temperaturzone nicht konstant, so meint der Begriff Temperatur einer Temperaturzone hier den (Zahlen)mittelwert der Temperatur der Festbettkatalysatorschüttung längs der Reaktionszone. Wesentlich ist dabei, dass die Temperierung der einzelnen Temperaturzonen im wesentlichen unabhängig voneinander erfolgt.

Da sowohl die heterogen katalysierte partielle Gasphasenoxidation von Propen zu Acrolein als auch die heterogen katalysierte partielle Gasphasenoxidation von Acrolein zu Acrylsäure eine ausgeprägt exotherme Reaktion ist, ist sowohl die Temperatur des Reaktionsgasgemisches 1 als auch die Temperatur des Reaktionsgasgemisches 2 beim reaktiven Durchgang durch die Festbettkatalysatorschüttung 1 bzw. Festbettkatalysatorschüttung 2 in der Regel von der Temperatur einer Temperaturzone verschieden. Sie liegt normalerweise oberhalb der Temperatur der Temperaturzone und durchläuft innerhalb einer Temperaturzone in der Regel ein Maximum (Heißpunktmaximum) oder fällt von einem Maximalwert ausgehend ab.

In der Regel wird beim erfindungsgemäßen Verfahren die Differenz $T^{maxA}$ - $T^{maxB}$ nicht mehr als 80°C betragen. Erfindungsgemäß bevorzugt beträgt $T^{maxA}$ - $T^{maxB}$ ≥ 3°C und ≤ 70°C. Ganz besonders bevorzugt beträgt $T^{maxA}$ - $T^{maxB}$ beim erfindungsgemäßen Verfahren ≥ 20°C und ≤ 60°C.

Die erfindungsgemäß geforderten Differenzen $T^{maxA}$ - $T^{maxB}$ stellen sich bei der Ausübung des erfindungsgemäßen Verfahren im Fall von eher niederen (≥ 90 Nl/l · h und ≤ 160 Nl/l · h) Propenbelastungen der Festbettkatalysatorschüttung 1 normalerweise dann ein, wenn einerseits sowohl die Temperatur der Reaktionszone A als auch die Temperatur der Reaktionszone B im Bereich von 290 bis 380°C liegt und andererseits die Differenz zwischen der Temperatur der Reaktionszone B ($T_B$) und der Temperatur der Reaktionszone A ($T_A$), d.h., $T_B$ - $T_A$, ≤ 0°C und ≥ -20°C oder ≥ -10°C bzw. ≤ 0°C und ≥ -5°C, oder häufig ≤ 0°C und ≥ -3°C beträgt.

Bei Ausübung des erfindungsgemäßen Verfahrens unter erhöhten Propenbelastungen (≥ 160 Nl/l · h und ≤ 300 Nl/l · h, bzw. ≤ 600 Nl/l · h) stellen sich die erfindungsgemäß geforderten Differenzen $T^{maxA}$ - $T^{maxB}$ normalerweise dann ein, wenn einerseits sowohl die Temperatur der Reaktionszone A als auch die Temperatur der Reaktionszone B im Bereich von 290 bis 380°C liegt und $T_B$ - $T_A$ ≥ 0°C und ≤ 50°C, oder ≥ 5°C und ≤ 45°C, oder ≥ 10°C und ≤ 40°C, oder ≥ 15°C und ≤ 30°C oder ≤ 35°C (z.B. 20°C oder 25°C) beträgt.

Die vorgenannte Aussage betreffend die Temperaturdifferenzen $T_B$ - $T_A$ gilt regelmäßig auch dann, wenn die Temperatur der Reaktionszone A im bevorzugten Bereich von 305 bis 365°C bzw. im besonders bevorzugten Bereich von 310 bis 340°C liegt.

Die Propenbelastung der Festbettkatalysatorschüttung 1 kann somit beim erfindungsgemäßen Verfahren z.B. ≥ 90 Nl/l · h und ≤ 300 Nl/l · h, oder ≥ 110 Nl/l · h und ≤ 280 Nl/l · h oder ≥ 130 Nl/l · h und ≤ 260 Nl/l · h, oder ≥ 150 Nl/l · h und ≤ 240 Nl/l · h, oder ≥ 170 Nl/l · h und ≤ 220 Nl/l · h, oder ≥ 190 Nl/l · h und ≤ 200 Nl/l · h betragen.

Erfindungsgemäß bevorzugt erstreckt sich die Temperaturzone A bis zu einem Umsatz des Propens von 50 bis 70 mol-% bzw. 60 bis 70 mol-%.

In der Regel wird beim erfindungsgemäßen Verfahren die Differenz $T^{maxC}$ - $T^{maxD}$ nicht mehr als 75°C betragen. Erfindungsgemäß bevorzugt beträgt $T^{maxC}$ - $T^{maxD}$ ≥ 3°C und ≤ 60°C. Ganz besonders bevorzugt beträgt $T^{maxG}$ - $T^{maxD}$ beim erfindungsgemäßen Verfahren ≥ 5°C und ≤ 40°C.

Die erfindungsgemäß geforderten Differenzen $T^{maxC}$ - $T^{maxD}$ stellen sich bei der Ausübung des erfindungsgemäßen Verfahren im Fall von eher niederen (≥ 70 Nl/l · h und ≤ 150 Nl/l - h) Acroleinbelastungen der Festbettkatalysatorschüttung 2 normalerweise dann ein, wenn einerseits sowohl die Temperatur der Reaktionszone C als auch die Temperatur der Reaktionszone D im Bereich von 230 bis 320°C liegt und andererseits die Differenz zwischen der Temperatur der Reaktionszone D ($T_D$) und der Temperatur der Reaktionszone C ($T_C$), d.h., $T_D$ - $T_C$, ≤ 0°C und ≥ -20°C oder ≥ -10°C bzw. ≤ 0°C und ≥ -5°C, oder häufig ≤ 0°C und ≥ -3°C beträgt.

Bei Ausübung des erfindungsgemäßen Verfahrens unter erhöhten Propenbelastungen (≥ 150 Nl/l · h und ≤ 300 Nl/l · h, bzw. ≤ 600 Nl/l · h) stellen sich die erfindungsgemäß geforderten Differenzen $T^{maxC}$ - $T^{maxD}$ normalerweise dann ein, wenn einerseits sowohl die Temperatur der Reaktionszone C als auch die Temperatur der Reaktionszone D im Bereich von 230 bis 320°C liegt und $T_D$ - $T_C$ ≥ 0°C und ≤ 40°C, oder ≥ 5°C und ≤ 35°C, bzw. 30°C, oder ≥ 10°C und ≤ 25°C, bzw. ≤ 20°C, oder ≤ 15°C beträgt.

Die vorgenannte Aussage betreffend die Temperaturdifferenzen $T_D$ - $T_C$ gilt regelmäßig auch dann, wenn die Temperatur der Reaktionszone C im bevorzugten Bereich von 250 bis 300°C bzw. im besonders bevorzugten Bereich von 260 bis 280°C liegt.

Die Acroleinbelastung der Festbettkatalysatorschüttung 2 kann somit beim erfindungsgemäßen Verfahren z.B. ≥ 70 Nl/l · h bzw. ≥ 90 Nl/l · h und ≤ 300 Nl/l. h, oder ≥ 110 Nl/l · h und ≤ 280 Nl/l · h oder ≥ 130 Nl/l · h und ≤ 260 Nl/l · h, oder ≥ 150 Nl/l · h und ≤ 240 Nl/l · h, oder ≥ 170 Nl/l · h und ≤ 220 Nl/l · h, oder ≥ 190 Nl/l · h und ≤ 200 Nl/l · h betragen.

Erfindungsgemäß bevorzugt erstreckt sich die Temperaturzone C bis zu einem Umsatz des Acroleins von 50 bis 85 mol-% bzw. 60 bis 85 mol-%.

Der Arbeitsdruck kann in beiden Reaktionsstufen des erfindungsgemäßen Verfahrens sowohl unterhalb von Normaldruck (z.B. bis zu 0,5 bar) als auch oberhalb von Normaldruck liegen. Typischerweise wird der Arbeitsdruck in den beiden Reaktionsstufen des erfindungsgemäßen Verfahrens bei Werten von 1 bis 5 bar, häufig 1 bis 3 bar liegen.

Normalerweise wird der Reaktionsdruck in keiner der beiden Reaktionsstufen 100 bar überschreiten.

In der Regel wird der auf den einfachen Durchgang der Festbettkatalysatorschüttung 1 bezogene Propenumsatz beim erfindungsgemäßen Verfahren ≥ 92 mol-% oder ≥ 94 mol-% betragen. Die Selektivität der Wertproduktbildung (Summe aus Acroleinbildung und Acrylsäurenebenproduktbildung) wird dabei bei in an sich bekannter Weise geeigneter Wahl der Festbettkatalysatorschüttung 1 regelmäßig ≥ 92 mol-%, oder ≥ 94 mol-%, häufig ≥ 95 mol-%, oder ≥ 96 mol-% bzw. ≥ 97 mol-% betragen.

In der Regel wird beim erfindungsgemäßen Verfahren die Acroleinbelastung der Festbettkatalysatorschüttung 2 ferner etwa 10 Nl/l · h, häufig etwa 20 bzw. 25 Nl/l · h unterhalb der Propenbelastung der Festbettkatalysatorschüttung 1 liegen. Dies ist primär darauf zurückzuführen, dass in der ersten Reaktionsstufe sowohl der Umsatz des Propens als auch die Selektivität der Acroleinbildung in der Regel keine 100 % erreichen.

In der Regel wird der auf den einfachen Durchgang der Festbettkatalysatorschüttung 2 bezogene Acroleinumsatz beim erfindungsgemäßen Verfahren ≥ 92 mol-%, oder ≥ 94 mol-%, oder ≥ 96 mol-%, oder ≥ 98 mol-% und häufig sogar ≥ 99 mol-% oder mehr betragen.

Bei in an sich bekannter Weise geeigneter Wahl der Festbettkatalysatorschüttungen 1, 2 kann beim erfindungsgemäßen Verfahren die über beide Reaktionsstufen bilanzierte Selektivität der Acrylsäurebildung, bezogen auf umgesetztes Propen, bei Werten ≥ 83 mol-%, häufig bei 85 mol-%, oder ≥ 88 mol-%, oft bei ≥ 90 mol-%, oder ≥ 93 mol-% liegen.

Das molare Verhältnis von $O_2:C_3H_6$ im Realctionsgasausgangsgemisch 1 muß erfindungsgemäß ≥ 1 betragen. Üblicherweise wird dieses Verhältnis bei Werten ≤ 3 liegen.

Häufig beträgt das molare Verhältnis von $O_2: C_3H_6$ im Reaktionsgasausgangsgemisch 1 erfindungsgemäß ≥ 1,5 und ≤ 2,0.

Das molare Verhältnis von $O_2: C_3H_4O$ im Reaktionsgasausgangsgemisch 2 beträgt erfindungsgemäß bevorzugt ebenfalls ≥ 1. Üblicherweise wird dieses Verhältnis ebenfalls bei Werten ≤ 3 liegen. Häufig wird das molare Verhältnis von $O_2$: Acrolein im Reaktionsgasausgangsgemisch 2 erfindungsgemäß 1 bis 2 bzw. 1 bis 1,5 betragen.

Als Katalysatoren für die Festbettkatalysatorschüttung 1 des erfindungsgemäßen Verfahrens kommen alle diejenigen in Betracht, deren Aktivmasse wenigstens ein Mo, Bi und Fe enthaltendes Multimetalloxid ist.

Dies sind insbesondere die Multimetalloxidaktivmassen der allgemeinen Formel I der DE-A 19955176, die Multimetalloxidaktivmassen der allgemeinen Formel I der DE-A 19948523, die Multimetalloxidaktivmassen der allgemeinen Formeln I, II und III der DE-A 10101695, die Multimetalloxidaktivmassen der allgemeinen Formeln I, II und III der DE-A 19948248 und die Multimetalloxidaktivmassen der allgemeinen Formeln I, II und III der DE-A 19955168 sowie die in der EP-A 700714 genannten Multimetalloxidaktivmassen.

Ferner eignen sich für die Festbettkatalysatorschüttung 1 die Mo, Bi und Fe enthaltenden Multimetalloxidkatalysatoren die in den Schriften DE-A 10046957, DE-A 10063162, DE-C 3338380, DE-A 19902562, EP-A 15565, DE-C 2380765, EP-A 807465, EP-A 279374, DE-A 3300044, EP-A 575897, US-A 4438217, DE-A 19855913, WO 98/24746, DE-A 19746210 (diejenigen der allgemeinen Formel II), JP-A 91/294239, EP-A 293224 und EP-A 700714 offenbart werden. Dies gilt insbesondere für die beispielhaften Ausführungsformen in diesen Schriften, unter denen jene der EP-A 15565, der EP-A 575897, der DE-A 19746210 und der DE-A 19855913 besonders bevorzugt werden. Besonders hervorzuheben sind in diesem Zusammenhang ein Katalysator gemäß Beispiel 1 c aus der EP-A 15565 sowie ein in entsprechender Weise herzustellender Katalysator, dessen Aktivmasse jedoch die Zusammensetzung $Mo_{12}Ni_{6,5}Zn_2Fe_2Bi_{1,0}O,_{0065}K_{0,06}O_x$ • 10 $SiO_2$ aufweist. Ferner sind hervorzuheben das Beispiel mit der laufenden Nr. 3 aus der DE-A 19855913 (Stöchiometrie: $Mo_{12}Co_7Fe_3Bi_{0,6}K_{0,08}Si_{1,6}Ox$) als Hohlzylindenrollkatalysator der Geometrie 5 mm x 3 mm x 2 mm bzw. 5 mm x 2 mm x 2 mm (jeweils Außendurchmesser x Höhe x Innendurchmesser) sowie der Multimetalloxid II - Vollkatalysator gemäß Beispiel 1 der DE-A 19746210. Ferner wären die Multimetalloxid-Katalysatoren der US-A 4438217 zu nennen. Letzteres gilt insbesondere dann, wenn diese eine Hohlzylinder-Geometrie der Ausmaße 5,5 mm x 3 mm x 3,5 mm, oder 5 mm x 2 mm x 2 mm, oder 5 mm x 3 mm x 2 mm, oder 6 mm x 3 mm x 3 mm, oder 7 mm x 3 mm x 4 mm (jeweils Außendurchmesser x Höhe x Innendurchmesser) aufweisen. Ebenso eignen sich die Multimetalloxidkatalysatoren und Geometrien der DE-A 10101695 bzw. WO 02/062737.

Weiterhin sind das Beispiel 1 aus der DE-A 10046957 (Stöchiometrie: $[Bi_2W_2O_9$ x $2WO_3]_{0,5}$ • $[Mo_{12}Co_{5,6}Fe_{2,94}Si_{1,59}K_{0,08}O_x]_1$) als Hohlzylinder (Ring) vollkatalysator der Geometrie 5 mm x 3 mm x 2 mm bzw. 5 mm x 2 mm x 2 mm (jeweils Außendurchmesser x Länge x Innendurchmesser), sowie die Schalenkatalysatoren 1, 2 und 3 aus der DE-A 10063162 (Stöchiometrie: $Mo_{12}Bi_{1,0}Fe_3Co_7Si_{1,6}K_{0,08}$), jedoch als ringförmige Schalenkatalysatoren entsprechender Schalendicke und auf Trägerringe der Geometrie 5 mm x 3 mm x 1,5 mm bzw. 7 mm x 3 mm x 1,5 mm (jeweils Außendurchmesser x Länge x Innendurchmesser) aufgebracht, geeignet.

Eine Vielzahl der für die Katalysatoren der Festbettkatalysatorschüttung 1 geeigneten Multimetalloxidaktivmassen lässt sich unter der allgemeinen Formel I

$$Mo_{12}Bi_aFe_bX^1_cX^2_dX^3_eX^4_fO_n \qquad (I),$$

in der die Variablen nachfolgende Bedeutung aufweisen:

$X^1 =$ Nickel und/oder Kobalt,
$X^2 =$ Thallium, ein Alkalimetall und/oder ein Erdalkalimetall,
$X^3 =$ Zink, Phosphor, Arsen, Bor, Antimon, Zinn, Cer, Blei und/oder Wolfram,
$X^4 =$ Silicium, Aluminium, Titan und/oder Zirkonium,

a = 0,5 bis 5,
b = 0,01 bis 5, vorzugsweise 2 bis 4,
c = 0 bis 10, vorzugsweise 3 bis 10,
d = 0 bis 2, vorzugsweise 0,02 bis 2,
e = 0 bis 8, vorzugsweise 0 bis 5,
f = 0 bis 10 und
n = eine Zahl, die durch die Wertigkeit und Häufigkeit der von Sauerstoff verschiedenen Elemente in I bestimmt wird,

subsummieren.

Sie sind in an sich bekannter Weise erhältlich (siehe z.B. die DE-A 4023239) und werden üblicherweise in Substanz zu Kugeln, Ringen oder Zylindern geformt oder auch in Gestalt von Schalenkatalysatoren, d.h., mit der Aktivmasse beschichteten vorgeformten, inerten Trägerkörpern, eingesetzt. Selbstverständlich können sie aber auch in Pulverform als Katalysatoren angewendet werden.

Prinzipiell können Aktivmassen der allgemeinen Formel I in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 650°C calciniert. Die Calcination kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemisch aus Inertgas, $NH_3$, CO und/oder $H_2$) erfolgen. Die Calcinationsdauer kann einige Minuten bis einige Stunden betragen und nimmt üblicherweise mit der Temperatur ab. Als Quellen für die elementaren Konstituenten der Multimetalloxidaktivmassen I kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Aminkomplexe, AmmoniumSalze und/oder Hydroxide in Betracht (Verbindungen wie $NH_4OH$, $(NH_4)_2CO_3$, $NH_4NO_3$, $NH_4CHO_2$, $CH_3COOH$, $NH_4CH_3CO_2$ und/oder Ammoniumoxalat, die spätestens beim späteren Calcinieren zu gasförmig entweichenden Verbindungen zerfallen und/oder zersetzt werden können, können in das innige Trockengemisch zusätzlich eingearbeitet werden).

Das innige Vermischen der Ausgangsverbindungen zur Herstellung von Multimetalloxidaktivmassen I kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wäßrige Masse getrocknet, wobei der Trocknungsprozeß vorzugsweise durch Sprühtrocknung der wäßrigen Mischung mit Austrittstemperaturen von 100 bis 150°C erfolgt.

Üblicherweise werden die Multimetalloxidaktivmassen der allgemeinen Formel I in der Festbettkatalysatorschüttung 1 nicht in Pulverform sondern zu bestimmten Katalysatorgeometrien geformt eingesetzt, wobei die Formgebung vor oder nach der abschließenden Calcination erfolgen kann. Beispielsweise können aus der Pulverform der Aktivmasse oder ihrer uncalcinierten und/oder partiell calcinierten Vodäufermasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Eine besonders günstige Hohlzylindergeometrie beträgt 5 mm x 3 mm x 2 mm (Außendurchmesser x Länge x Innendurchmesser), insbesondere im Fall von Vollkatalysatoren.

Selbstverständlich kann die Formgebung der pulverförmigen Aktivmasse oder ihrer pulverförmigen, noch nicht und/oder partiell calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z.B. aus der DE-A 2909671, der EP-A 293859 oder aus der EP-A 714700 bekannt ist. Zweckmäßigerweise wird zur Beschichtung der Trägerkörper die aufzubringende Pulvermasse befeuchtet und nach dem Aufbringen, z.B. mittels heißer Luft, wieder getrocknet. Die Schichtdicke der auf den Trägerkörper aufgebrachten Pulvermasse wird zweckmäßigerweise im Bereich 10 bis 1000 μm, bevorzugt im Bereich 50 bis 500 μm und besonders bevorzugt im Bereich 150 bis 250 μm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Sie verhalten sich bezüglich der dem erfindungsgemäßen Verfahren in der ersten Reaktionsstufe unterliegenden Zielrealition in der Regel im wesentlichen inert. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Geeignet ist die Verwendung von im wesentlichen unporösen, oberflächenrauhen, kugelförmigen Trägern aus Steatit (z.B. Steatit C220 der Fa. CeramTec), deren Durchmesser 1 bis 8 mm, bevorzugt 4 bis 5 mm beträgt. Geeignet ist aber auch die Verwendung von Zylindern als Trägerkörper, deren Länge 2 bis 10 mm und deren Außendurchmesser 4. bis 10 mm beträgt. Im Fall von erfindungsgemäß geeigneten Ringen als Trägerkörper liegt die Wanddicke darüber hinaus üblicherweise bei 1 bis 4 mm. Erfindungsgemäß bevorzugt zu verwendende ringförmige Trägerkörper besitzen eine Länge von 2 bis 6 mm, einen Außendurchmesser von 4 bis 8 mm und eine Wanddicke von 1 bis 2 mm. Erfindungsgemäß geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Für die Katalysatoren der ersten Reaktionsstufe geeignete Multimetalloxidaktivmassen sind ferner Massen der allgemeinen Formel II

$$[Y^1_{a'}Y^2_{b'}O_{x'}]_p[Y^3_{c'}Y^4_{d'}Y^5_{e'}Y^6_{f'}Y^7_{g'}Y^2_{h'}O_{y'}]_q \qquad (II),$$

in der die Variablen folgende Bedeutung haben:

$Y^1 =$ nur Wismut oder Wismut und wenigstens eines der Elemente Tellur, Antimon, Zinn und Kupfer,
$Y^2 =$ Molybdän oder Molybdän und Wolfram,
$Y^3 =$ ein Alkalimetall, Thallium und/oder Samarium,
$Y^4 =$ ein Erdalkalimetall, Nickel, Kobalt, Kupfer, Mangan, Zink, Zinn, Cadmium und/oder Quecksilber,
$Y^5 =$ Eisen oder Eisen und wenigstens eines der Elemente Chrom und Cer,
$Y^6 =$ Phosphor, Arsen, Bor und/oder Antimon,
$Y^7 =$ ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,

$a' =$ 0,01 bis 8,
$b' =$ 0,1 bis 30,
$c' =$ 0 bis 4,
$d' =$ 0 bis 20,
$e' >$ 0 bis 20,
$f' =$ 0 bis 6,
$g' =$ 0 bis 15,
$h'$ = 8 bis 16,
$x',y'=$ Zahlen, die durch die Wertigkeit und Häufigkeit der von Sauerstoff verschiedenen Elemente in II bestimmt werden und
$p,q =$ Zahlen, deren Verhältnis p/q 0,1 bis 10 beträgt,

enthaltend dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung $Y^1_{a'}Y^2_{b'}O_{x'}$, deren Größtdurchmesser (längste durch den Schwerpunkt des Bereichs gehende Verbindungsstrecke zweier auf der Oberfläche (Grenzfläche) des Bereichs befindlicher Punkte) 1 nm bis 100 μm, häufig 10 nm bis 500 nm oder 1 μm bis 50 bzw. 25 μm, beträgt.

Besonders vorteilhafte erfindungsgemäße Multimetalloxidmassen II sind solche, in denen $Y^1$ nur Wismut ist.

Unter diesen werden wiederum jene bevorzugt, die der allgemeinen Formel III

$$[Bi_{a''}Z^2_{b''}O_{x'}]_{p''} [Z^2_{12}Z^3_{c''}Z^4_{d''}Fe_{e''}Z^5_{f''}Z^6_{g''}Z^7_{h''}O_{y''}]_{q''} \qquad (III)$$

in der die Varianten folgende Bedeutung haben:

$Z^2 =$ Molybdän oder Molybdän und Wolfram,
$Z^3 =$ Nickel und/oder Kobalt,
$Z^4 =$ Thallium, ein Alkalimetall und/oder ein Erdalkalimetall,
$Z^5 =$ Phosphor, Arsen, Bor, Antimon, Zinn, Cer und/oder Blei,
$Z^6 =$ Silicium, Aluminium, Titan und/oder Zirkonium,
$Z^7 =$ Kupfer, Silber und/oder Gold,

a" = 0,1 bis 1,
b" = 0,2 bis 2,
c" = 3 bis 10,
d" = 0,02 bis 2,
e" = 0,01 bis 5, vorzugsweise 0,1 bis 3,
f' = 0 bis 5,
g" = 0 bis 10,
h" = 0 bis 1,
x",y"= Zahlen, die durch die Wertigkeit und Häufigkeit der von Sauerstoff verschiedenen Element in III bestimmt werden,
p",q"= Zahlen, deren Verhältnis p"/q" 0,1 bis 5, vorzugsweise 0,5 bis 2 beträgt,

entsprechen, wobei diejenigen Massen III ganz besonders bevorzugt werden, in denen $Z^2_{b"}$ = (Wolfram)$_{b"}$ und $Z^2_{12}$ = (Molybdän)$_{12}$ ist.

Ferner ist es von Vorteil, wenn wenigstens 25 mol-% (bevorzugt wenigstens 50 mol-% und besonders bevorzugt wenigstens 100 mol-%) des gesamten Anteils $[Y^1_a, Y^2_b, O_{x'}]_p$ ($[Bi_{a"}Z^2_{b"}O_{x"}]_{p"}$) der erfindungsgemäß geeigneten Multimetalloxidmassen II (Multimetalloxidmassen III) in den erfindungsgemäß geeigneten Multimetallo;;idmassen II (Multimetalloxidmassen III) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung $Y^1_a, Y^2_b, O_x$, $[Bi_{a"}Z^2_{b"}O_{x"}]$) vorliegen, deren Größtdurchmesser im Bereich 1 nm bis 100 $\mu$m liegt.

Hinsichtlich der Formgebung gilt bezüglich Multimetalloxidmassen II-Katalysatoren das bei den Multimetalloxidmassen I-Katalysatoren Gesagte.

Die Herstellung von Multimetalloxidmassen II-Aktivrnassen ist z.B. in der EP-A 575897 sowie in der DE-A 19855913 beschrieben.

Die vorstehend empfohlenen inerten Trägermaterialien kommen u.a. auch als inerte Materialien zur Verdünnung und/oder Abgrenzung des entsprechenden Katalysatorfestbetts, bzw. als deren schützende Vorschüttung in Betracht.

Für die Katalysatoren der Festbettkatalysatorschüttung 2 kommen prinzipiell alle Mo und V enthaltenden Multimetalloxidmassen als Aktivmassen in Betracht, z.B. jene der DE-A 10046928.

Eine Vielzahl derselben, z.B. diejenigen der DE-A 19815281, lässt sich unter der allgemeinen Formel IV

$$Mo_{12}V_aX^1_bX^2_cX^3_dX^4_eX^5_fX^6_gO_n \qquad (IV),$$

in der die Variablen folgende Bedeutung haben:

$X^1$ = W, Nb, Ta, Cr und/oder Ce,
$X^2$ = Cu, Ni, Co, Fe, Mn und/oder Zn,
$X^3$ = Sb und/oder Bi,
$X^4$ = eines oder mehrere Alkalimetalle,
$X^5$ = eines oder mehrere Erdalkalimetalle,
$X^6$ = Si, Al, Ti und/oder Zr,

a = 1 bis 6,
b = 0,2 bis 4,
c = 0,5 bis 18,
d = 0 bis 40,
e = 0 bis 2,
f = 0 bis 4,
g = 0 bis 40 und
n = eine Zahl, die durch die Wertigkeit und Häufigkeit der von Sauerstoff verschiedenen Elemente in IV bestimmt wird,

subsummieren.

Erfindungsgemäß bevorzugte Ausführungsformen innerhalb der aktiven Multimetalloxide IV sind jene, die von den nachfolgenden Bedeutungen der Variablen der allgemeinen Formel IV erfasst werden:

$X^1$ = W, Nb, und/oder Cr,
$X^2$ = Cu, Ni, Co, und/oder Fe,
$X^3$ = Sb,
$X^4$ = Na und/oder K,

$X^5 =$   Ca, Sr und/oder Ba,
$X^6 =$   Si, Al, und/oder Ti,

$a =$   1,5 bis 5,
$b =$   0,5 bis 2,
$c =$   0,5 bis 3,
$d =$   0 bis 2,
$e =$   0 bis 0,2,
$f =$   0 bis 1 und
$n =$   eine Zahl, die durch die Wertigkeit und Häufigkeit der von Sauerstoff verschiedenen Elemente in IV bestimmt wird.

Erfindungsgemäß ganz besonders bevorzugte Multimetalloxide IV sind jedoch jene der allgemeinen Formel V

$$Mo_{12}V_{a'}Y1_{b'}Y2_{c'}Y5_{f'}Y6_{g'}O_{n'} \qquad (V),$$

mit

$Y^1 =$   W und/oder Nb,
$Y^2 =$   Cu und/oder Ni,
$Y^5 =$   Ca und/oder Sr,
$Y^6 =$   Si und/oder Al,

$a' =$   2 bis 4,
$b' =$   1 bis 1,5,
$c' =$   1 bis 3,
$f' =$   0 bis 0,5
$g' =$   0 bis 8 und
$n' =$   eine Zahl, die durch die Wertigkeit und Häufigkeit der von Sauerstoff verschiedenen Elementen in V bestimmt wird.

Die erfindungsgemäß geeigneten Multimetalloxidaktivmassen (IV) sind in an sich bekannter, z.B. in der DE-A 4335973 oder in der EP-A 714700 offenbarter, Weise erhältlich.
Prinzipiell können für die Katalysatoren der Festbettkatalysatorschüttung 2 geeignete Multimetalloxidaktivmassen, insbesondere solche der allgemeinen Formel IV, in einfacher Weise dadurch hergestellt werden, daß man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 600°C calciniert. Die Calcination kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemische aus Inertgas und reduzierenden Gasen wie $H_2$, $NH_3$, CO, Methan und/oder Acrolein oder die genannten reduzierend wirkenden Gase für sich) durchgeführt werden. Die Calcinationsdauer kann einige Minuten bis einige Stunden betragen und nimmt üblicherweise mit der Temperatur ab. Als Quellen für die elementaren Konstituenten der Multimetalloxidaktivmassen IV kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.
Das innige Vermischen der Ausgangsverbindungen zur Herstellung von Multimetalloxidmassen IV kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form.
Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150°C erfolgt.
Die resultierenden Multimetalloxidmassen, insbesondere jene der allgemeinen Formel IV, werden in der Regel nicht in Pulverform sondern zu bestimmten Katalysatorgeometrien geformt in der Festbettkatalysatorschüttung 2 eingesetzt werden, wobei die Formgebung vor oder nach der abschließenden Calcination erfolgen kann. Beispielsweise können aus der Pulverform der Aktivmasse oder ihrer uncalcinierten Vorläufermasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Selbstverständlich kann die Formgebung der pulverförmigen Aktivmasse oder ihrer pulverförmigen, noch nicht calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z.B. aus der DE-A 2909671, der EP-A 293859 oder aus der EP-A 714700 bekannt ist.

Zweckmäßigerweise wird zur Beschichtung der Trägerkörper die aufzubringende Pulvermasse befeuchtet und nach dem Aufbringen, z.B. mittels heißer Luft, wieder getrocknet. Die Schichtdicke der auf den Trägerkörper aufgebrachten Pulvermasse wird zweckmäßigerweise im Bereich 10 bis 1000 $\mu$m, bevorzugt im Bereich 50 bis 500 $\mu$m und besonders bevorzugt im Bereich 150 bis 250 $\mu$m liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder mit Splittauflage, bevorzugt werden. Geeignet ist die Verwendung von im wesentlichen unporösen, oberflächenrauhen, kugelförmigen Trägern aus Steatit (z.B. Steatit C220 der Fa. CeramTec), deren Durchmesser 1 bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von Ringen als Trägerkörper liegt die Wanddicke darüber hinaus üblicherweise bei 1 bis 4 mm. Bevorzugt zu verwendende ringförmige Trägerkörper besitzen eine Länge von 2 bis 6 mm, einen Außendurchmesser von 4 bis 8 mm und eine Wanddicke von 1 bis 2 mm. Geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Günstige für die Katalysatoren der Festbettkatalysatorschüttung 2 zu verwendende Multimetalloxidaktivmassen sind ferner Massen der allgemeinen Formel VI,

$$[D]_p[E]_q \qquad (VI),$$

in der die Variablen folgende Bedeutung haben:

$D = Mo_{12}V_{a''}Z^1_{b''}Z^2_{c''}Z^3_{d''}Z^4_{e''}Z^5_{f''}Z^6_{g''}O_{x''}$,
$E = Z^7_{12}Cu_{h''}H_{i''}O_{y''}$,
$Z^1 = $ W, Nb, Ta, Cr und/oder Ce,
$Z^2 = $ Cu, Ni, Co, Fe, Mn und/oder Zn,
$Z^3 = $ Sb und/oder Bi,
$Z^4 = $ Li, Na, K, Rb, Cs und/oder H
$Z^5 = $ Mg, Ca, Sr und/oder Ba,
$Z^6 = $ Si, Al, Ti und/oder Zr,
$Z^7 = $ Mo, W, V, Nb und/oder Ta, vorzugsweise Mo und/oder W

$a'' = $ 1 bis 8,
$b'' = $ 0,2 bis 5,
$c'' = $ 0 bis 23,
$d'' = $ 0 bis 50,
$e'' = $ 0 bis 2,
$f = $ 0 bis 5,
$g'' = $ 0 bis 50,
$h'' = $ 4 bis 30,
$i'' = $ 0 bis 20 und
$x'', y'' = $ Zahlen, die durch die Wertigkeit und Häufigkeit der von Sauerstoff verschiedenen Element in VI bestimmt werden und
$p, q = $ von Null verschiedene Zahlen, deren Verhältnis p/q 160:1 bis 1:1 beträgt,

und die dadurch erhältlich sind, dass man eine Multimetalloxidmasse E

$$Z^7_{12}Cu_{h''}H_{i''}O_{y''} \qquad (E),$$

in feinteiliger Form getrennt vorbildet (Ausgangsmasse 1) und anschließend die vorgebildete feste Ausgangsmasse 1 in eine wässrige Lösung, eine wässrige Suspension oder in ein feinteiliges Trockengemisch von Quellen der Elemente Mo, V, $Z^1$, $Z^2$, $Z^3$, $Z^4$, $Z^5$, $Z^6$, die die vorgenannten Elemente in der Stöchiometrie D

$$Mo_{12}V_{a''}Z^1_{b''}Z^2_{c''}Z^3_{d''}Z^4_{e''}Z^5_{f''}Z^6_{g''} \qquad (D),$$

enthält (Ausgangsmasse 2), im gewünschten Mengenverhältnis p:q einarbeitet, die dabei gegebenenfalls resultierende wässrige Mischung trocknet, und die so gegebene trockene Vorläufermasse vor oder nach ihrer Trocknung zur gewünschten Katalysatorgeometrie bei Temperaturen von 250 bis 600°C calciniert.

Bevorzugt sind die Multimetalloxidmassen VI, bei denen die Einarbeitung der vorgebildeten festen Ausgangsmasse 1 in eine wässrige Ausgangsmasse 2 bei einer Temperatur < 70°C erfolgt. Eine detaillierte Beschreibung der Herstellung von Multimetalloxidmassen VI-Katalysatoren enthalten z.B. die EP-A 668104, die DE-A 19736105, die DE-A 10046928, die DE-A 19740493 und die DE-A 19528646.

Hinsichtlich der Formgebung gilt bezüglich Multimetalloxidmassen VI-Katalysatoren das bei den Multimetalloxidmassen IV-Katalysatoren Gesagte.

Darüber hinaus eignen sich als für die Katalysatoren der Festbettkatalysatorschüttung 2 geeignete Multimetalloxidmassen jene der DE-A 19815281, insbesondere alle beispielhaften Ausführungsformen aus dieser Schrift. Hinsichtlich der Formgebung gilt das vorstehend Gesagte.

Für die Festbettkatalysatorschüttung 2 des erfindungsgemäßen Verfahrens besonders geeignete Katalysatoren sind die Schalenkatalysatoren S1 (Stöchiometrie: $Mo_{12}V_3W_{1,2}CU_{2,4}O_n$) und S7 (Stöchiometrie: $Mo_{12}V_3W_{1,2}Cu_{1,6}Ni_{0,8}O_n$) aus der DE-A 4442346 mit einem Aktivmassenanteil von 27 Gew.-% und einer Schalendicke von 230 μm, der Schalenkatalysator aus Herstellungsbeispiel 5 der DE-A 10046928 (Stöchiometrie: $Mo_{12}V_3W_{1,2}Cu_{2,4}O_n$) mit einem Aktivmassenanteil von 20 Gew.-%, die Schalenkatalysatoren gemäß den Beispielen 1 bis 5 aus der DE-A 19815281, jedoch ebenso wie die vorstehend genannten Schalenkatalysatoren für die zweite Reaktionsstufe auf Trägerringe der Geometrie 7 mm x 3 mm x 4 mm (Außendurchmesser x Länge x Innendurchmesser) mit einem Aktivmassenanteil von 20 Gew.-% (bezogen auf die Gesamtmasse des Schalenkatalysators) aufgebracht, sowie ein Schalenkatalysator mit zweiphasiger Aktivmasse der Stöchiometrie $(Mo_{10,4}V_3W_{1,2}O_x)$ $(CuMo_{0,5}W_{0,5}O_4)_{1,6}$, und hergestellt gemäß der DE-A 19736105 und einem Aktivmassenanteil von 20 Gew.-% auf den vorgenannten 7 mm x 3 mm x 4 mm Träger aufgebracht.

Die vorstehend für die zweite Reaktionsstufe empfohlenen Katalysatoren sind für die zwei Reaktionsstufen aber auch dann geeignet, wenn man alles beibehält und nur die Trägergeometrie auf 5 mm x 3 mm x 1,5 mm abändert (Außendurchmesser x Länge x Innendurchmesser). Ferner können die genannten Multimetalloxide aber auch in Form der entsprechenden Vollkatalysatorringe in der zweiten Reaktionsstufe eingesetzt werden.

Erfindungsgemäß wesentlich ist, dass die Festbettkatalysatorschüttung 1 aus wenigstens zwei räumlich aufeinander folgenden Festbettkatalysatorschüttungszonen besteht, wobei die volumenspezifische Aktivität innerhalb einer Festbettkatalysatorschüttungszone im wesentlichen konstant ist und in Strömungsrichtung des Reaktionsgasgemisches 1 beim Übergang von einer Festbettkatalysatorschüttungszone in eine andere Festbettkatalysatorschüttungszone sprunghaft zunimmt.

Die volumenspezifische (d.h., die auf die Einheit der jeweiligen Schüttungsvolumens normierte) Aktivität einer Festbettkatalysatorschüttungszone kann nun in über die Festbettkatalysatorschüttungszone im wesentlichen konstanter Weise dadurch eingestellt werden, dass man von einer Grundmenge einheitlich hergestellter Katalysatorformkörper ausgeht (ihre Schüttung entspricht der maximal erzielbaren volumenspezifischen Aktivität) und diese in der jeweiligen Festbettkatalysatorschüttungszone mit sich bezüglich der heterogen katalysierten partiellen Gasphasenoxidation im wesentlichen inert verhaltenden Formkörpern (Verdünnungsformkörper) homogen verdünnt. Je höher der Anteil der Verdünnungsformkörper gewählt wird, desto geringer ist die in einem bestimmten Volumen der Schüttung enthaltene Aktivmasse bzw. Katalysatoraktivität. Als Materialien für solche inerten Verdünnungsformkörper kommen prinzipiell alle diejenigen in Betracht, die sich auch als Trägermaterial für erfindungsgemäß geeignete Schalenkatalysatoren eignen.

Als solche Materialien kommen z.B. poröse oder unporöse Aluminiumoxide, Siliciumdioxid, Thoriumdioxid, Zirkondioxid, Siliziumcarbid, Silikate wie Magnesium -oder Aluminiumsilikat oder der bereits erwähnte Steatit (z.B. Steatit C-220 der Fa. Ceram Tec) in Betracht.

Die Geometrie solcher inerter Verdünnungsformkörper kann im Prinzip beliebig sein. D.h., es können beispielsweise Kugeln, Polygone, Vollzylinder oder auch Ringe sein. Erfindungsgemäß bevorzugt wird man als inerte Verdünnungsformkörper solche wählen, deren Geometrie derjenigen der mit ihnen zu verdünnenden Katalysatorformkörper entspricht.

Erfindungsgemäß günstig ist es, wenn sich die chemische Zusammensetzung der verwendeten Aktivmasse über die gesamte Festbettkatalysatorschüttung 1 nicht verändert. D.h., die für einen einzelnen Katalysatorformkörper verwendete Aktivmasse kann zwar ein Gemisch aus verschiedenen, die Elemente Mo, Fe und Bi enthaltenden, Multimetalloxiden sein, für alle Katalysatorformkörper der Festbettkatalysatorschüttung 1 ist dann jedoch das gleiche Gemisch zu verwenden.

Eine in Strömungsrichtung des Reaktionsgasgemisches 1 über die Festbettkatalysatorschüttung zonenweise zunehmende volumenspezifische Aktivität lässt sich für das erfindungsgemäße Verfahren somit in einfacher Weise z.B. dadurch einstellen, dass man die Schüttung in einer ersten Festbettkatalysatorschüttungszone mit einem hohen Anteil an inerten Verdünnungsformkörpern bezogen auf eine Sorte von Katalysatorformkörpem beginnt, und dann diesen Anteil an Verdünnungsformkörpern in Strömungsrichtung zonenweise verringert.

Eine zonenweise Zunahme der volumenspezifischen Aktivität ist aber auch z.B. dadurch möglich, dass man bei gleich bleibender Geometrie und Aktivmassenart eines Schalenkatalysatorformkörpers zonenweise die Dicke der auf dem Träger aufgebrachten Aktivmassenschicht erhöht oder in einem Gemisch aus Schalenkatalysatoren mit gleicher Geometrie aber mit unterschiedlichem Gewichtsanteil der Aktivmasse zonenweise den Anteil an Katalysatorformkörpern mit höherem Aktivmassenanteil steigert. Alternativ kann man auch die Aktivmassen selbst verdünnen, in dem man bei der Aktivmassenherstellung z.B. in das zu calcinierende Trockengemisch aus Ausgangsverbindungen inerte verdünnend wirkende Materialien wie hochgebranntes Silicimdioxid einarbeitet. Unterschiedliche Zusatzmengen an verdünnend wirkendem Material führen automatisch zu unterschiedlichen Aktivitäten. Je mehr verdünnend wirkendes Material zugesetzt wird, desto geringer wird die resultierende Aktivität sein. Analoge Wirkung lässt sich z.B. auch dadurch erzielen, dass man bei Mischungen aus Vollkatalysatoren und aus Schalenkatalysatoren (bei identischer Aktivmasse) in entsprechender Weise das Mischungsverhältnis verändert. Des weiteren lässt sich eine Variation der volumenspezifischen Aktivität durch den Einsatz von Katalysatorgeometrien mit unterschiedlicher Schüttdichte erzielen (z.B. bei Vollkatalysatoren mit identischer Aktivmassenzusammensetzung der verschiedenen Geometrien). Selbstredend lassen sich die beschriebenen Varianten auch kombiniert anwenden.

Natürlich können für die Festbettkatalysatorschüttung 1 aber auch Mischungen aus Katalysatoren mit chemisch unterschiedlicher Aktivmassenzusammensetzung und als Folge dieser verschiedenen Zusammensetzung unterschiedlicher Aktivität verwendet werden. Diese Mischungen können wiederum zonenweise in ihrer Zusammensetzung variiert und/oder mit unterschiedlichen Mengen inerter Verdünnungsformkörper verdünnt werden.

Vorab und/oder im Anschluß an die Festbettkatalysatorschüttung 1 können sich ausschließlich aus Inertmaterial (z.B. nur Verdünnungsformkörpern) bestehende Schüttungen befinden (sie werden in dieser Schrift begrifflich nicht der Festbettkatalysatorschüttung 1 zugerechnet, da sie keine Formkörper enthalten, die Multimetalloxidaktivmasse aufweisen). Dabei können die für die Inertschüttung verwendeten Verdünnungsformkörper die gleiche Geometrie wie die in der Festbettkatalysatorschüttung 1 verwendeten Katalysatorformkörper aufweisen. Die Geometrie der für die Inertschüttung verwendeten Verdünnungsformkörper kann aber auch von der vorgenannten Geometrie der Katalysatorformkörper verschieden sein (z.B. kugelförmig anstelle ringförmig).

Häufig weisen die für solche Inertschüttungen verwendeten Formkörper die ringförmige Geometrie 7 mm x 7 mm x4 mm (Außendurchmesser x Länge x Innendurchmesser) oder die kugelförmige Geometrie mit dem Durchmesser d = 4 - 5 mm auf. Die Temperaturzonen A und B können sich beim erfindungsgemäßen Verfahren auch auf die 1-nertschüttungen erstrecken. Erfindungsgemäß vorteilhaft erfassen sowohl die Temperaturzone A als auch die Temperaturzone B jeweils nicht mehr als drei Festbettkatalysatorschüttungszonen (erfindungsgemäß zwingend wird wenigstens eine Festbettkatalysatorschüttungszone von beiden Temperaturzonen erfasst).

Erfindungsgemäß besonders vorteilhaft umfasst die gesamte Festbettkatalysatorschüttung nicht mehr als fünf, zweckmäßig nicht mehr als vier bzw. drei Festbettkatalysatorschüttungszonen.

Beim Übergang von einer Festbettkatalysatorschüttungszone in eine andere Festbettkatalysatorschüttungszone (in Strömungsrichtung des Reaktionsgasgemisches 1) der Festbettkatalysatorschüttung 1 sollte (bei einheitlicher Aktivmasse über die gesamte Festbettkatalysatorschüttung 1) die volumenspezifische Aktivmasse (d.h., das Gewicht der im Einheitsschüttungsvolumen enthaltenen Multimetalloxidaktivmasse) erfindungsgemäß zweckmäßig um wenigstens 5 Gew.-%, bevorzugt um wenigstens 10 Gew.-% zunehmen (dies gilt insbesondere auch bei einheitlichen Katalysatorformkörpern über die gesamte Festbettkatalysatorschüttung 1). In der Regel wird diese Zunahme beim erfindungsgemäßen Verfahren nicht mehr als 50 Gew.-%, meist nicht mehr als 40 Gew.-% betragen. Ferner sollte bei einheitlicher Aktivmasse über die gesamte Festbettkatalysatorschüttung 1 der Unterschied in der volumenspezifischen Aktivmasse derjenigen Festbettkatalysatorschüttungszone mit der geringsten volumenspezifischen Aktivität und derjenigen Festbettkatalysatorschüttungszone mit der höchsten volumenspezifischen Aktivität erfindungsgemäß nicht mehr als 50 Gew.-%, vorzugsweise nicht mehr als 40 Gew.-% und in der Regel nicht mehr als 30 Gew.-% betragen.

Häufig wird beim erfindungsgemäßen Verfahren die Festbettkatalysatorschüttung 1 aus nur zwei Festbettkatalysatorschüttungszonen bestehen.

Erfindungsgemäß bevorzugt ist die in Strömungsrichtung des Reaktionsgasgemisches 1 letzte Festbettkatalysatorschüttungszone der Festbettkatalysatorschüttung 1 unverdünnt. D.h., sie besteht vorzugsweise ausschließlich aus Katalysatorformkörpern. Im Bedarfsfall kann sie auch aus einer Schüttung aus Katalysatorformkörpem bestehen, deren volumenspezifische Aktivität z.B. durch Verdünnung mit Inertmaterial abgesenkt, z.B. um 10 %, ist

Besteht die Festbettkatalysatorschüttung 1 nur aus zwei Festbettkatalysatorschüttungszonen, ist es erfindungsgemäß in der Regel vorteilhaft (wie ganz allgemein beim erfindungsgemäßen Verfahren), wenn die Festbettkatalysatorschüttungszone mit der höchsten volumenspezifischen Aktivität nicht bis in die Temperaturzone A hineinragt (insbesondere dann, wenn in der Temperaturzone A und in der Temperaturzone B die Temperierung mittels eines fließenden Wärmeträgers erfolgt, der jeweils im Gegenstrom zum Reaktionsgasgemisch strömt). D.h., in günstiger Weise wird die Festbettkatalysatorschüttungszone mit der geringeren volumenspezifischen Aktivität in die Temperaturzone B hineinragen und die Festbettkatalysatorschüttungszone mit der höheren volumenspezifischen Aktivität in der Temperaturzone B beginnen und enden (d.h., hinter dem Übergang von der Temperaturzone A in die Temperaturzone B ihren Anfang haben).

Besteht die Festbettkatalysatorschüttung 1 nur aus drei Festbettkatalysatorschüttungszonen, ist es erfindungsgemäß in der Regel gleichfalls vorteilhaft, wenn die Festbettkatalysatorschüttungszone mit der höheren volumenspezifischen Aktivität nicht bis in die Temperaturzone A hineinragt sondern in der Temperaturzone B beginnt und endet, d.h., hinter dem Übergang von der Temperaturzone A in die Temperaturzone B ihren Anfang hat (insbesondere dann, wenn in der Temperaturzone A und in der Temperaturzone B die Temperierung mittels eines fließenden Wärmeträgers erfolgt, der jeweils im Gegenstrom zum Reaktionsgasgemisch strömt). D.h., normalerweise wird in diesem Fall die Festbettkatalysatorschüttungszone mit der zweithöchsten volumenspezifischen Aktivität sowohl in die Temperaturzone A als auch in die Temperaturzone B hineinragen.

Besteht die Festbettkatalysatorschüttung 1 aus vier Festbettkatalysatorschüttungszonen, ist es erfindungsgemäß in der Regel vorteilhaft, wenn die Festbettkatalysatorschüttungszone mit der dritthöchsten volumenspezifischen Aktivität sowohl in die Temperaturzone A als auch in die Temperaturzone B hineinragt (insbesondere dann, wenn in der Temperaturzone A und in der Temperaturzone B die Temperierung mittels eines fließenden Wärmeträgers erfolgt, der jeweils im Gegenstrom zum Reaktionsgasgemisch strömt).

Im Fall einer Gleichstromführung von Reaktionsgasgemisch und Wärmeträgern in den Temperaturzone A und B kann es vorteilhaft sein, wenn beim erfindungsgemäßen Verfahren innerhalb der Festbettkatalysatorschüttung 1 die Festbettkatalysatorschüttungszone mit der höchstens volumenspezifischen Aktivität in die Temperaturzone A hineinragt.

Generell lässt sich die volumenspezifische Aktivität zwischen zwei Festbettkatalysatorschüttungszonen einer Festbettkatalysatorschüttung 1 experimentell in einfacher Weise so differenzieren, dass unter identischen Randbedingungen (vorzugsweise den Bedingungen des ins Auge gefassten Verfahrens) über Festbettkatalysatorschüttungen derselben Länge, aber jeweils der Zusammensetzung der jeweiligen Festbettkatalysatorschüttungszone entsprechend, das gleich Propen enthaltende Reaktionsgasgemisch geführt wird. Die höhere umgesetzte Menge an Propen weist die höhere volumenspezifische Aktivität aus.

Beträgt die Gesamtlänge der Festbettkatalysatorschüttung 1 $L^1$, ist es erfindungsgemäß vorteilhaft, wenn sich im Bereich $X^1 \pm L^1 \dfrac{4}{100}$ bzw. im Bereich $X^1 \pm L^1 \dfrac{3}{100}$ bzw.

im Bereich $X^1 \pm L^1 \dfrac{2}{100}$ kein Übergang von einer Festbettkatalysatorschüttungszone in eine andere Festbettkatalysatorschüttungszone befindet, wobei X der Ort (die Stelle) innerhalb der Festbettkatalysatorschüttung 1 ist, an dem der Übergang von der Temperaturzone A in die Temperaturzone B erfolgt.

Erfindungsgemäß bevorzugt ist beim erfindungsgemäßen Verfahren die Festbettkatalysatorschüttung 1 in Strömungsrichtung des Reaktionsgasgemisches wie folgt strukturiert.

Zunächst auf einer Länge von 10 bis 60 %, bevorzugt 10 bis 50 %, besonders bevorzugt 20 bis 40 % und ganz besonders bevorzugt 25 bis 35 % (d.h. z.B. auf einer Länge von 0,70 bis 1,50 m, bevorzugt 0,90 bis 1,20 m), jeweils der Gesamtlänge der Festbettkatalysatorschüttung 1, ein homogenes Gemisch aus Katalysatorformkörpern und Verdünnungsformkörpern (wobei beide vorzugsweise im wesentlichen die gleiche Geometrie aufweisen), wobei der Gewichtsanteil der Verdünnungsformkörper (die Massendichten von Katalysatorformkörpern und von Verdünnungsformkörpern unterscheiden sich in der Regel nur geringfügig) normalerweise 5 bis 40 Gew.-%, oder 10 bis 40 Gew.-%, oder 20 bis 40 Gew.-%, oder 25 bis 35 Gew.-% beträgt. Im Anschluss an diese erste Zone der Festbettkatalysatorschüttung befindet sich dann erfindungsgemäß vorteilhaft bis zum Ende der Länge der Festbettkatalysatorschüttung (d.h., z.B. auf einer Länge von 2,00 bis 3,00 m, bevorzugt 2,50 bis 3,00 m) entweder eine nur in geringerem Umfang (als in der ersten Zone) verdünnte Schüttung der Katalysatorformkörper, oder, ganz besonders bevorzugt, eine alleinige (unverdünnte) Schüttung derselben Katalysatorformkörper, die auch in der ersten Zone verwendet worden sind. Das Vorgenannte trifft insbesondere dann zu, wenn in der Festbettkatalysatorschüttung als Katalysatorformkörper Vollkatalysatorringe oder Schalenkatalysatorringe (insbesondere jene, die in dieser Schrift als bevorzugt genannt werden) eingesetzt werden. Mit Vorteil weisen im Rahmen der vorgenannten Strukturierung sowohl die Katalysatorformkörper als auch die Verdünnungsformkörper beim erfindungsgemäßen Verfahren im wesentlichen die Ringgeometrie 5 mm x 3 mm x 2 mm (Außendurchmesser x Länge x Innendurchmesser) auf.

Das Vorgenannte trifft auch dann zu, wenn anstelle inerter Verdünnungsformkörper Schalenkatalysatorformkörper verwendet werden, deren Aktivmassenanteil um 2 bis 15 Gew.-% Punkte tiefer liegt, als der Aktivmassenanteil der am Ende der Festbettkatalysatorschüttung 1 gegebenenfalls verwendeten Schalenkatalysatorformkörper.

Eine reine Inertmaterialschüttung, deren Länge, bezogen auf die Länge der Festbettkatalysatorschüttung 1, zweckmäßig 5 bis 20 % beträgt, leitet in Strömungsrichtung des Reaktionsgasgemisches in der Regel die Festbettkatalysatorschüttung 1 ein. Sie wird normalerweise als Aufheizzone für das Reaktionsgasgemisch genutzt.

Erfindungsgemäß vorteilhaft erstreckt sich nun bei den vorgenannten Festbettkatalysatorschüttungen 1 die Festbettkatalysatorschüttungzone mit der geringeren volumenspezifischen Aktivität noch auf 5 bis 20 %, häufig auf 5 bis 15 % ihrer Länge in die Temperaturzone B.

Erfindungsgemäß zweckmäßig erstreckt sich die Temperaturzone A auch auf eine zur Festbettkatalysatorschüttung 1 gegebenenfalls angewandte Vorschüttung aus Inertmaterial.

Erfindungsgemäß wesentlich ist ferner, dass die Festbettkatalysatorschüttung 2 aus wenigstens zwei räumlich aufeinander folgenden Festbettkatalysatorschüttungszonen besteht, wobei die volumenspezifische Aktivität innerhalb einer Festbettkatalysatorschüttungszone im wesentlichen konstant ist und in Strömungsrichtung das Reaktionsgasgemisches beim Übergang von einer Festbettkatalysatorschüttungszone in eine andere Festbettkatalysatorschüttungszone sprunghaft zunimmt.

Die volumenspezifische (d.h., die auf die Einheit der jeweiligen Schüttungsvolumens normierte) Aktivität einer Festbettkatalysatorschüttungszone kann nun in über die Festbettkatalysatorschüttungszone im wesentlichen konstanter Weise dadurch eingestellt werden, dass man von einer Grundmenge einheitlich hergestellter Katalysatorformkörper ausgeht (ihre Schüttung entspricht der maximal erzielbaren volumenspezifischen Aktivität) und diese in der jeweiligen Festbettkatalysatorschüttungszone mit sich bezüglich der heterogen katalysierten partiellen Gasphasenoxidation im wesentlichen inert verhaltenden Formkörpern (Verdünnungsformkörper) homogen verdünnt. Je höher der Anteil der Verdünnungsformkörper gewählt wird, desto geringer ist die in einem bestimmten Volumen der Schüttung enthaltene Aktivmasse bzw. Katalysatoraktivität. Als Materialien für solche inerten Verdünnungsformkörper kommen prinzipiell alle diejenigen in Betracht, die sich auch als Trägermaterial für erfindungsgemäß geeignete Schalenkatalysatoren eignen.

Als solche Materialien kommen z.B. poröse oder unporöse Aluminiumoxide, Siliciumdioxid, Thoriumdioxid, Zirkondioxid, Siliciumcarbid, Silikate wie Magnesium- oder Aluminiumsilikat oder der bereits erwähnte Steatit (z.B. Steatit C-220 der Fa. Cerm Tec) in Betracht.

Die Geometrie solcher inerter Verdünnungsformkörper kann im Prinzip beliebig sein. D.h., es können beispielsweise Kugeln, Polygone, Vollzylinder oder auch Ringe sein. Erfindungsgemäß bevorzugt wird man als inerte Verdünnungsformkörper solche wählen, deren Geometrie derjenigen der mit ihnen zu verdünnenden Katalysatorformkörper entspricht.

Erfindungsgemäß günstig ist es, wenn sich die chemische Zusammensetzung der verwendeten Aktivmasse über die gesamte Festbettkatalysatorschüttung 2 nicht verändert. D.h., die für einen einzelnen Katalysatorformkörper verwendete Aktivmasse kann zwar ein Gemisch aus verschiedenen, die Elemente Mo und V enthaltenden, Multimetalloxiden sein, für alle Katalysatorformkörper der Festbettkatalysatorschüttung 2 ist dann jedoch das gleiche Gemisch zu verwenden.

Eine in Strömungsrichtung des Reaktionsgasgemisches 2 über die Festbettkatalysatorschüttung 2 zonenweise zunehmende volumenspezifische Aktivität lässt sich für das erfindungsgemäße Verfahren somit in einfacher Weise z.B. dadurch einstellen, dass man die Schüttung in einer ersten Festbettkatalysatorschüttungszone mit einem hohen Anteil an inerten Verdünnungsformkörpern bezogen auf eine Sorte von Katalysatorformkörpern beginnt, und dann diesen Anteil an Verdünnungsformkörpern in Strömungsrichtung zonenweise verringert.

Eine zonenweise Zunahme der volumenspezifischen Aktivität ist aber auch z.B. dadurch möglich, dass man bei gleich bleibender Geometrie und Aktivmassenart eines Schalenkatalysatorformkörpers zonenweise die Dicke der auf dem Träger aufgebrachten Aktivmassenschicht erhöht oder in einem Gemisch aus Schalenkatalysatoren mit gleicher Geometrie aber mit unterschiedlichem Gewichtsanteil der Aktivmasse zonenweise den Anteil an Katalysatorformkörpern mit höherem Aktivmassengewichtsanteil steigert. Alternativ kann man auch die Aktivmassen selbst verdünnen, in dem man bei der Aktivmassenherstellung z.B. in das zu calcinierende Trockengemisch aus Ausgangsverbindungen inerte verdünnend wirkende Materialien wie hochgebranntes Siliciumdioxid einarbeitet. Unterschiedliche Zusatzmengen an verdünnend wirkendem Material führen automatisch zu unterschiedlichen Aktivitäten. Je mehr verdünnend wirkendes Material zugesetzt wird, desto geringer wird die resultierende Aktivität sein. Analoge Wirkung lässt sich z.B. auch dadurch erzielen, dass man bei Mischungen aus Vollkatalysatoren und aus Schalenkatalysatoren (bei identischer Aktivmasse) in entsprechender Weise das Mischungsverhältnis verändert. Des weiteren lässt sich eine Variation der volumenspezifischen Aktivität durch den Einsatz von Katalysatorgeometrien mit unterschiedlicher Schüttdichte erzielen (z.B. bei Vollkatalysatoren mit identischer Aktivmassenzusammensetzung der verschiedenen Geometrien). Selbstredend lassen sich die beschriebenen Varianten auch kombiniert anwenden.

Natürlich können für die Festbettkatalysatorschüttung 2 aber auch Mischungen aus Katalysatoren mit chemisch unterschiedlicher Aktivmassenzusammensetzung und als Folge dieser verschieden Zusammensetzung unterschiedlicher Aktivität verwendet werden. Diese Mischungen können wiederum zonenweise in ihrer Zusammensetzung variiert und/oder mit unterschiedlichen Mengen inerter Verdünnungsformkörper verdünnt werden.

Vorab und/oder im Anschluss an die Festbettkatalysatorschüttung 2 können sich ausschließlich aus Inertmaterial (z.B. nur Verdünnungsformkörpern) bestehende Schüttungen befinden (sie werden in dieser Schicht begrifflich nicht der Festbettkatalysatorschüttung 2 zugerechnet, da sie keine Formkörper enthalten, die Multimetalloxidaktivmasse aufweisen). Dabei können die für die Inertschüttung verwendeten Verdünnungsformkörper die gleich Geometrie wie die in der Festbettkatalysatorschüttung verwendeten Verdünnungsformkörper aufweisen. Die Geometrie der für die Inertschüttung verwendeten Verdünnungsformkörper kann aber auch von der vorgenannten Geometrie der Katalysatorformkörper verschieden sein (z.B. kugelförmige anstatt ringförmig).

Häufig weisen die für solche Inertschüttungen verwendeten Formkörper die ringförmige Geometrie 7 mm x 7 mm x 4 mm (Außendurchmesser x Länge x Innendurchmesser) oder die kugelförmige Geometrie mit dem Durchmesser d = 4 - 5 mm auf. Die Temperaturzone C und D können sich beim erfindungsgemäßen Verfahren auch auf die Inertschüttungen erstrecken. Erfindungsgemäß vorteilhaft erfassen sowohl die Temperaturzone C als auch die Temperaturzone D jeweils nicht mehr als drei Festbettkatalysatorschüttungszonen, (erfindungsgemäß zwingend wird wenigstens eine Festbettkatalysatorschüttungszone von beiden Temperaturzonen erfasst).

Erfindungsgemäß besonders vorteilhaft umfasst die gesamte Festbettkatalysatorschüttung 2 nicht mehr als fünf, zweckmäßig nicht mehr als vier bzw. drei Festbettkatalysatorschüttungszonen.

Beim Übergang von einer Festbettkatalysatorschüttungszone in eine andere Festbettkatalysatorschüttungszone (in Strömungsrichtung des Reaktionsgasgemisches 2) sollte (bei einheitlicher Aktivmasse über die gesamte Festbettkatalysatorschüttung 2) die volumenspezifische Aktivmasse (d.h., das Gewicht der im Einheitsschüttungsvolumen enthaltenen Multimetalloxidaktivmasse) erfindungsgemäß zweckmäßig um wenigstens 5 Gew.-%, bevorzugt um wenigstens 10 Gew.-% zunehmen (dies gilt insbesondere auch bei einheitlichen Katalysatorformkörpern über die gesamte Festbettkatalysatorschüttung 2). In der Regel wird diese Zunahme beim erfindungsgemäßen Verfahren nicht mehr als 50 Gew.-%, meist nicht mehr als 40 Gew.-% betragen. Ferner sollte bei einheitlicher Aktivmasse über die gesamte Festbettkatalysatorschüttung 2 der Unterschied in der volumenspezifischen Aktivmasse derjenigen Festbettkatalysatorschüttungszone mit der geringsten volumenspezifischen Aktivität und derjenigen Festbettkatalysatorschüttungszone mit der höchsten volumenspezifischen Aktivität erfindungsgemäß nicht mehr als 50 Gew.-%, vorzugsweise nicht mehr als 40 Gew.-% und besonders bevorzugt nicht mehr als 30 Gew.-% betragen.

Häufig wird beim erfindungsgemäßen Verfahren die Festbettkatalysatorschüttung 2 aus nur zwei Festbettkatalysatorschüttungszonen bestehen.

Erfindungsgemäß bevorzugt ist die in Strömungsrichtung des Realctionsgasgemisches letzte Festbettlzatalysatorschüttungszone der Festbettkataiysatorschüttung 2 unverdünnt. D.h., sie besteht vorzugsweise ausschließlich aus Katalysatorformkörpern. Im Bedarfsfall kann sie auch aus einer Schüttung aus Katalysatorformkörpern bestehen, deren volumenspezifische Aktivität z.B. durch Verdünnung mit Inertmaterial abgesenkt, z.B. um 10 %, ist. Besteht die Festbettkatalysatorschüttung 2 nur aus zwei Festbettkatalysatorschüttungszonen, ist es erfindungsgemäß in der Regel vorteilhaft (wie ganz allgemein beim erfindungsgemäßen Verfahren), wenn die Festbettkatalysatorschüttungszone mit der höchsten volumenspezifischen Aktivität bis in die Temperaturzone C hineinragt (insbesondere dann, wenn in der Temperaturzone C und in der Temperaturzone D die Temperierung mittels eines fließenden Wärmeträgers erfolgt, der jeweils im Gegenstrom zum Reaktionsgasgemisch strömt).

Besteht die Festbettkatalysatorschüttung 2 nur aus drei Festbettkatalysatorschüttungszonen, ist es erfindungsgemäß in der Regel gleichfalls vorteilhaft, wenn die Festbettkatalysatorschüttungszone mit der höchsten volumenspezifischen Aktivität bis in die Temperaturzone C hineinragt (insbesondere dann, wenn in der Temperaturzone C und in der Temperaturzone D die Temperierung mittels eines fließenden Wärmeträgers erfolgt, der jeweils im Gegenstrom zum Reaktionsgasgemisch strömt).

Besteht die Festbettkatalysatorschüttung 2 aus vier Festbettkatalysatorschüttungszonen, ist es erfindungsgemäß in der Regel vorteilhaft, wenn die Festbettkatalysatorschüttungszone mit der zweithöchsten volumenspezifischen Aktivität sowohl in die Temperaturzone C als auch in die Temperaturzone D hineinragt (insbesondere dann, wenn in der Temperaturzone C und in der Temperaturzone D die Temperierung mittels eines fließenden Wärmeträgers erfolgt, der jeweils im Gegenstrom zum Reaktionsgasgemisch strömt).

Im Fall einer Gleichstromführung von Reaktionsgasgemisch und Wärmeträgern in den Temperaturzonen C und D kann es erfindungsgemäß vorteilhaft sein, wenn innerhalb der Festbettkatalysatorschüttung 2 die Festbettkatalysatorschüttungszone mit der höchsten volumenspezifischen Aktivität nicht in die Temperaturzone C hineinragt, sondern erst hinter dem Übergang von der Temperaturzone C in die Temperaturzone D ihren Anfang hat.

Die volumenspezifische Aktivität zwischen zwei Festbettkatalysatorschüttungszonen innerhalb der Festbettkatalysatorschüttung 2 läßt sich experimentell in einfacher Weise so differenzieren, dass unter identischen Randbedingungen (vorzugsweise den Bedingungen des ins Auge gefassten Verfahrens) über Festbettkatalysatorschüttungen der selben Länge, aber jeweils der Zusammensetzung der jeweiligen Festbetikatalysatorschüttungszone entsprechend, das gleich Acrolein enthaltende Reaktionsgasausgangsgemisch geführt wird. Die höhere umgesetzte Menge an Acrolein weist die höhere volumenspezifische Aktivität aus.

Beträgt die Gesamtlänge der Festbettkatalysatorschüttung 2 L$^2$, ist es erfindungsgemäß vorteilhaft, wenn sich im Bereich $X^2 \pm L^2 \dfrac{4}{100}$ bzw. im Bereich $X^2 \pm L^2 \dfrac{3}{100}$ bzw. im Bereich $X^2 \pm L^2 \dfrac{2}{100}$ kein Übergang von einer Festbettkatalysatorschüttungszone in eine andere Festbettkatalysatorschüttungszone befindet, wobei X der Ort innerhalb der Festbettkatalysatorschüttung 2 ist, an dem der Übergang von der Temperaturzone C in die Temperaturzone D erfolgt.

Erfindungsgemäß bevorzugt ist beim erfindungsgemäßen Verfahren die Festbettkatalysatorschüttung 2 in Strömungsrichtung des Reaktionsgasgemisches wie folgt strukturiert.

Zunächst auf einer Länge von 10 bis 60 %, bevorzugt 10 bis 50 %, besonders bevorzugt 20 bis 40 % und ganz besonders bevorzugt 25 bis 35 % (d.h., z.B. auf einer Länge von 0,70 bis 1,50 m, bevorzugt 0,90 bis 1,20 m), jeweils der Gesamtlänge der Festbettkatalysatorschüttung 2, ein homogenes Gemisch oder zwei (mit abnehmender Verdünnung) aufeinander folgende homogene Gemische aus Katalysatorformkörpem und Verdünnungsformkörpern (wobei beide vorzugsweise im wesentlichen die gleich Geometrie aufweisen), wobei der Anteil der Verdünnungsformkörper so bemessen ist, dass die volumenspezifische Aktivmasse, bezogen auf eine nur aus den Katalysatorformkörpern bestehende Schüttung, um 10 bis 50 Gew.-%, vorzugsweise 20 bis 45 Gew.-% und besonders bevorzugt 25 bis 35 Gew.-% abgesenkt ist. Im Anschluss an diese erste bzw. an diese beiden ersten Zonen befindet sich dann erfindungsgemäß vorteilhaft bis zum Ende der Länge der Festbettkatalysatorschüttung 2 (d.h., z.B. auf einer Länge von 2,00 bis 3,00 m, bevorzugt 2,50 bis 3,00 m) entweder eine nur in geringerem Umfang (als in der ersten bzw. in den ersten beiden Zonen) verdünnte Schüttung der Katalysatorformkörper, oder, ganz besonders bevorzugt, eine alleinige Schüttung derselben Katalysatorformkörper, die auch in den ersten Zonen verwendet worden sind.

Das Vorgenannte trifft insbesondere dann zu, wenn in der Festbettkatalysatorschüttung 2 als Katalysatorformkörper Schalenkatalysatorringe oder Schalenkatalysatorkugeln eingesetzt werden (insbesondere jene, die in dieser Schrift als bevorzugt angeführt werden). Mit Vorteil weisen im Rahmen der vorgenannten Strukturierung sowohl die Katalysatorformkörper bzw. deren Trägerringe als auch die Verdünnungsformkörper beim erfindungsgemäßen Verfahren im wesentlichen die Ringgeometrie 7 mm x 3 mm x 4 mm (Außendurchmesser x Länge x Innendurchmesser) auf.

Das oben Genannte trifft auch dann zu, wenn anstelle inerter Verdünnungsformkörper Schalenkatalysatorformkörper verwendet werden, deren Aktivmassenanteil um 2 bis 15 Gew.-% Punkte tiefer liegt, als der Aktivmassenanteil der Schalenkatalysatorformkörper am Ende der Festbettkatalysatorschüttung.

Eine reine Inertmaterialschüttung, deren Länge, bezogen auf die Länge der Festbettkatalysatorschüttung 2, zweckmäßig 5 bis 20 % beträgt, leitet in Strömungsrichtung des Reaktionsgasgemisches in der Regel die Festbettkatalysatorschüttung 2 ein. Sie dient normalerweise dem Zweck der Temperierung des Reaktionsgasgemisches.

Erfindungsgemäß vorteilhaft erstreckt sich nun bei den vorgenannten Festbettkatalysatorschüttungen 2 die Temperaturzone C (die sich erfindungsgemäß vorteilhaft auch auf die Vorschüttung aus Inertmaterial erstreckt) noch auf 5 bis 20 %, häufig auf 5 bis 15 % der Länge der in Strömungsrichtung des Reaktionsgasgemisches 2 letzten (volumenspezifisch aktivsten) Festbettkatalysatorschüttungszone der Festbettkatalysatorschüttung 2.

In anwendungstechnisch zweckmäßiger Weise erfolgt die Durchführung der ersten Reaktionsstufe des erfindungsgemäßen Verfahrens in einem Zweizonenrohrbündelreaktor, wie er z.B. in den DE-A's 19910508, 19948523, 19910506 und 19948241 beschrieben ist. Eine bevorzugte Variante eines erfindungsgemäß einsetzbaren Zweizonenrohrbündelreaktors offenbart die DE-C 2830765. Aber auch die in der DE-C 2513405, der US-A 3147084, der DE-A 2201528, der EP-A 383224 und der DE-A 2903218 offenbarten Zweizonenrohrbündelreaktoren sind für eine Durchführung der ersten Reaktionsstufe des erfindungsgemäßen Verfahrens geeignet.

D.h., in einfachster Weise befindet sich die erfindungsgemäß zu verwendende Festbettkatalysatorschüttung 1 (eventuell mit vor- und/oder nachangeordneten Inertschüttungen) in den Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert erfindungsgemäß eine Temperaturzone. D.h., in einfachster Weise umströmt z.B. ein Salzbad A denjenigen Abschnitt der Rohre (die Temperaturzone A), in welchem sich die oxidative Umsetzung des Propens (beim einfachen Durchgang) bis zum Erreichen eines Umsatzes im Bereich von 40 bis 80 mol-% vollzieht und ein Salzbad B umströmt den Abschnitt der Rohre (die Reaktionszone B), in welchem sich die oxidative Anschlussumsetzung des Propens (beim einfachen Durchgang) bis zum Erreichen einesUmsatzwertes von wenigstens 90 mol-% vollzieht (bei Bedarf können sich an die erfindungsgemäß anzuwendenden Temperaturzonen A,B weitere Reaktionszonen anschließen, die auf individuellen Temperaturen gehalten werden).

Anwendungstechnisch zweckmäßig umfasst die erste Reaktionsstufe des erfindungsgemäßen Verfahrens keine weiteren Temperaturzonen. D.h., das Salzbad B umströmt zweckmäßig den Abschnitt der Rohre, in welchem sich die oxidative Anschlussumsetzung des Propens (beim einfachen Durchgang) bis zu einem Umsatzwert ≥ 90 mol-%, oder ≥ 92 mol-% oder ≥ 94 mol-% oder mehr vollzieht.

Üblicherweise liegt der Beginn der Temperaturzone B hinter dem Heißpunktmaximum der Temperaturzone A.

Die beiden Salzbäder A,B können erfindungsgemäß relativ zur Strömungsrichtung des durch die Reaktionsrohre strömenden Reaktionsgasgemisches im Gleichstrom oder im Gegenstrom durch den die Reaktionsrohre umgebenden Raum geführt werden. Selbstverständlich kann erfindungsgemäß auch in der Temperaturzone A eine Gleichströmung und in der Temperaturzone B eine Gegenströmung (oder umgekehrt) angewandt werden.

Selbstverständlich kann man in allen vorgenannten Fallkonstellationen innerhalb der jeweiligen Temperaturzone der, relativ zu den Reaktionsrohren, erfolgenden Parallelströmung der Salzschmelze noch eine Querströmung überlagern, so dass die einzelne Reaktionszone einem wie in der EP-A 700714 oder in der EP-A 700893 beschriebenen Rohrbündelreaktor entspricht und insgesamt im Längsschnitt durch das Kontaktrohrbündel ein mäanderförmiger Strömungsverlauf des Wärmeaustauschmittels resultiert.

Zweckmäßigerweise wird beim erfindungsgemäßen Verfahren das Reaktionsgasausgangsgemisch 1 der Festbettkatalysatorschüttung 1 auf die Reaktionstemperatur vorerwärmt zugeführt.

Üblicherweise sind in den Zweizonenrohrbündelreaktoren die Kontaktrohre aus ferritischem Stahl geferügt und weisen in typischer Weise eine Wanddicke von 1 bis 3 mm auf. Ihr Innendurchmesser beträgt in der Regel 20 bis 30 mm, häufig 21 bis 26 mm. Ihre Länge beträgt zweckmäßig 2 bis 4 m, bevorzugt 2,5 bis 3,5 m. In jeder Temperaturzone belegt die Festbettkatalysatorschüttung 1 wenigstens 60 % bzw. wenigstens 75 %, oder wenigstens 90 % der Länge der Zone. Die gegebenenfalls verbleibende Restlänge wird gegebenenfalls von einer Inertschüttung belegt. Anwendungstechnisch zweckmäßig beläuft sich die im Rohrbündelbehälter untergebrachte Anzahl an Kontaktrohren auf wenigstens 5000, vorzugsweise auf wenigstens 10000. Häufig beträgt die Anzahl der im Reaktionsbehälter untergebrachten Kontaktrohre 15000 bis 30000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet (bevorzugt 6 äquidistante Nachbarrohre pro Kontaktrohr), wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. z.B. EP-B 468290).

Als Wärmeaustauschmittel eignen sich auch für die Zweizonenfahrweise insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

In der Regel wird bei allen vorstehend erwähnten Konstellationen der Stromführung in den Zweizonenrohrbündelreaktoren die Fließgeschwindigkeit innerhalb der beiden erforderlichen Wärmeaustauschmittelkreisläufen so gewählt, dass die Temperatur des Wärmeaustauschmittels von der Eintrittsstelle in die Temperaturzone bis zur Austrittstelle aus der Temperaturzone (bedingt durch die Exothermie der Reaktion) um 0 bis 15°C ansteigt. D.h., das vorgenannte $\Delta T$ kann erfindungsgemäß 1 bis 10°C, oder 2 bis 8°C oder 3 bis 6°C betragen.

Die Eintrittstemperatur des Wärmeaustauschmittels in die Temperaturzone A liegt erfindungsgemäß normalerweise im Bereich von 290 bis 380°C, bevorzugt im Bereich von 305 bis 365°C und besonders bevorzugt im Bereich von 310 bis 340°C bzw. bei 330°C. Bei Propenbelastungen der Festbettkatalysatorschüttung 1 von $\geq$ 90 NI/l·h und $\leq$ 160 NI/l·h wird die Eintrittstemperatur des Wärmeaustauschmittels in die Temperaturzone B erfindungsgemäß ebenfalls im Bereich von 290 bis 380°C, gleichzeitig aber normalerweise erfindungsgemäß zweckmäßig $\geq$ 0°C bis $\leq$ 20°C, oder $\leq$ 10°C, bzw. $\geq$ 0°C und $\leq$ 5°C, oder häufig $\geq$ 0°C und $\leq$ 3°C unterhalb der Eintrittstemperatur des in die Temperaturzone A eintretenden Wärmeaustauschmittels liegen. Bei Propenbelastungen der Festbettkatalysatorschüttung 2 von $\geq$ 160 NI/l·h und (in der Regel) $\leq$ 300 NI/l·h (bzw. 600 NI/l·h) wird die Eintrittstemperatur des Wärmeaustauschmittels in die Temperaturzone B erfindungsgemäß ebenfalls im Bereich von 290 bis 380°C, aber normalerweise erfindungsgemäß zweckmäßig $\geq$ 0°C bis $\leq$ 50°C, oder $\geq$ 5°C und $\leq$ 45°C, oder $\geq$ 10°C und $\leq$ 40°C, oder $\geq$ 15°C und $\leq$ 30°C oder $\leq$ 35°C (z.B. 20°C oder 25°C) oberhalb der Eintrittstemperatur des in die Temperaturzone A eintretenden Wärmeaustauschmittels liegen.

Es sei an dieser Stelle auch noch einmal darauf hingewiesen, dass für eine Durchführung der Reaktionsstufe 1 des erfindungsgemäßen Verfahrens insbesondere auch der in der DE-AS 2201528 beschriebene Zweizonenrohrbündelreaktortyp verwendet werden kann, der die Möglichkeit beinhaltet, vom heißeren Wärmeaustauschmittel der Temperaturzone B eine Teilmenge an die Temperaturzone A abzuführen, um gegebenenfalls ein Anwärmen eines kalten Reaktionsgasausgangsgemisches oder eines kalten Kreisgases zu bewirken. Ferner kann die Rohrbündelcharakteristik innerhalb einer individuellen Temperaturzone wie in der EP-A 382098 beschrieben gestaltet werden.

Erfindungsgemäß hat es sich als zweckmäßig erwiesen, das die erste Reaktionsstufe verlassende Produktgasgemisch vor dem Eintritt in die zweite Reaktionsstufe auf direkte und/oder indirekte Weise abzukühlen, um so eine Nachvollverbrennung von Teilen des in der ersten Reaktionsstufe gebildeten Acroleins zu unterdrücken. Üblicherweise wird dazu zwischen die beiden Reaktionsstufen ein Nachkühler geschaltet. Dies kann im einfachsten Fall ein indirekter Rohrbündelwärmeüberträger sein. Das Produktgasgemisch wird dabei in der Regel durch die Rohre geführt und um die Rohre wird ein Wärmetauschermedium geführt, dessen Art der für die Rohrbündelreaktoren empfohlenen Wärmetauschermedien entsprechen kann. Mit Vorteil ist das Rohrinnere mit inerten Füllkörpern (z.B. Spiralen aus Edelstahl, Ringe aus Steatit, Kugeln aus Steatit etc.) gefüllt. Selbige verbessern den Wärmeaustausch und fangen gegebenenfalls aus der Festbettkatalysatorschüttung der ersten Reaktionsstufe sublimierendes Molybdäntrioxid vor einem Eintritt desselben in die zweite Reaktionsstufe ab. Es ist von Vorteil, wenn der Nachkühler aus mit Zinksilicattarbe beschichtetem rostfreiem Stahl gefertigt ist.

In der Regel wird der auf den einfachen Durchgang bezogene Propenumsatz beim erfindungsgemäßen Verfahren in der ersten Reaktionsstufe ≥ 92 mol-% oder ≥ 94 mol-% betragen. Die in der ersten Reaktionsstufe bei einfachem Durchgang resultierende Selektivität der Acroleinbildung sowie der Acrylsäurenebenproduktbildung wird dabei erfindungsgemäß zusammen regelmäßig ≥ 92 mol-% oder ≥ 94 mol-%, häufig ≥ 95 mol-% oder ≥ 96 mol-% bzw. ≥ 97 mol-% betragen.

Als Quelle für den in der ersten Reaktionsstufe erforderlichen molekularen Sauerstoff kommt sowohl Luft, als auch an molekularem Stickstoff entreicherte Luft (z.B. ≥ 90 Vol.% $O_2$, ≤ 10 Vol.-% $N_2$) in Betracht.

Anwendungstechnisch zweckmäßig wird das Produktgasgemisch der ersten Reaktionsstufe im bereits erwähnten Nachkühler auf eine Temperatur von 210 bis 290°C, häufig 230 bis 280°C oder 250 bis 270°C abgekühlt. Dabei kann die Abkühlung des Produktgasgemisches der ersten Reaktionsstufe durchaus auf Temperaturen erfolgen, die unterhalb der Temperatur der zweiten Reaktionsstufe liegen. Die beschriebene Nachkühlung ist jedoch keineswegs zwingend und kann insbesondere dann in aller Regel entfallen, wenn der Weg des Produktgasgemisches von der ersten Reaktionsstufe in die zweite Reaktionsstufe kurz gehalten wird. Üblicherweise wird das erfindungsgemäße Verfahren ferner so verwirklicht, dass man den Sauerstoffbedarf in der zweiten Reaktionsstufe nicht bereits durch einen entsprechend hohen Sauerstoffgehalt des Reaktionsgasausgangsgemisches 1 deckt, sondern den benötigten Sauerstoff im Bereich zwischen erster und zweiter Reaktionsstufe zugibt ("Sekundärgaszusatz") Dies kann vor, während, nach und/ oder zur Nachkühlung erfolgen. Als Quelle für den in der zweiten Reaktionsstufe erforderlichen molekularen Sauerstoff kommen sowohl reiner Sauerstoff als auch Gemische aus Sauerstoff und Inertgas, z.B. Luft oder an molekularem Stickstoff entreicherte Luft (z.B. ≥ 90 Vol-% $O_2$, ≤ 10 Vol-% $N_2$) in Betracht. Die Zugabe der Sauerstoffquelle erfolgt regelmäßig in auf den Reaktionsdruck komprimierter Form. Selbstredend kann beim erfindungsgemäßen Verfahren der Sauerstoffbedarf in der zweiten Reaktionsstufe bereits durch einen entsprechend hohen Sauerstoffbedarf in der ersten Reaktionsstufe gedeckt werden. Natürlich kann bei Bedarf als Sekundärgas auch ein inertes Verdünnungsgas zugesetzt werden.

Wie die Durchführung der ersten Reaktionsstufe erfolgt auch die Durchführung der zweiten Reaktionsstufe des erfindungsgemäßen Verfahrens in anwendungstechnisch zweckmäßiger Weise in einem Zweizonenrohrbündelreaktor, wie er für die erste Reaktionsstufe bereits beschrieben wurde. Die Ausführungen hinsichtlich des Zweizonenrohrbündelreaktors für die erste Reaktionsstufe gelten deshalb auch für den Zweizonenrohrbündelreaktor für die zweite Reaktionsstufe.

D.h., in einfacher Weise befindet sich die erfindungsgemäß zu verwendende Festbettkatalysatorschüttung 2 (gegebenenfalls einschließlich der Inertschüttungen) in den Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert erfindungsgemäß eine Temperaturzone.

D.h., in einfacher Weise umströmt z.B. ein Salzbad C diejenigen Abschnitte der Rohre (die Temperaturzone C), in welchem sich die oxidative Umsetzung des Acroleins (beim einfachen Durchgang) bis zum Erreichen eines Umsatzwertes im Bereich von 45 bis 85 mol-% (bevorzugt 50 bis 85 mol-%, besonders bevorzugt 60 bis 85 mol-%) vollzieht und ein Salzbad D umströmt den Abschnitt der Rohre (die Temperaturzone D), in welchem sich die oxidative Anschlussumsetzung des Acroleins (beim einfachen Durchgang) bis zum Erreichen eines Umsatzwertes von wenigstens 90 mol-% vollzieht (bei Bedarf können sich an die erfindungsgemäß anzuwendenden Temperaturzonen C, D weitere Temperaturzonen anschließen, die auf individuellen Temperaturen gehalten werden).

Anwendungstechnisch zweckmäßig umfasst die Reaktionsstufe 2 des erfindungsgemäßen Verfahrens keine weiteren Temperaturzonen. D.h., das Salzbad D umströmt zweckmäßig den Abschnitt der Rohre, in welchem sich die oxidative Anschlussumsetzung des Acroleins (beim einfachen Durchgang) bis zu einem Umsatzwert von ≥ 92 mol-%, oder ≥ 94 mol-% oder ≥ 96 mol-% oder ≥ 98 mol-% und häufig sogar ≥ 99 mol-% oder mehr vollzieht.

Üblicherweise liegt der Beginn der Temperaturzone D hinter dem Heißpunktmaximum der Temperaturzone C

Die beiden Salzbäder C, D können erfindungsgemäß relativ zur Strömungsrichtung des durch die Reaktionsrohre strömenden Reaktiongasgemisches im Gleichstrom oder im Gegenstrom durch den die Reaktionsrohre umgebenden Raum geführt werden. Selbstverständlich kann erfindungsgemäß auch in der Temperaturzone C eine Gleichströmung und in der Temperaturzone D eine Gegenströmung (oder umgekehrt) angewandt werden.

Selbstredend kann man in allen vorgenannten Fallkonstellationen innerhalb der jeweiligen Temperaturzone der, relativ zu den Reaktionsrohren, erfolgenden Parallelströmung der Salzschmelze noch eine Querströmung überlagern, so dass die einzelne Reaktionszone einem wie in der EP-A 700714 oder in der EP-A 700893 beschriebenen Rohrbündelreaktor entspricht und insgesamt im Längsschnitt durch das Kontaktrohrbündel ein mäanderförmiger Strömungsverlauf des Wärmeaustauschmittels resultiert.

Üblicherweise sind in den vorgenannten Zweizonen-Rohrbündelreaktoren für die zweite Reaktionsstufe die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 21 bis 26 mm. Ihre Länge beträgt zweckmäßig 3 bis 4, bevorzugt 3,5 m. In jeder Temperaturzone belegt die Festbettkatalysatorschüttung 2 wenigstens 60 %, bzw. wenigstens 75 %, oder wenigstens 90 % der Länge der Zone. Die gegebenenfalls verbleibende Restlänge wird gegebenenfalls von einer Inertschüttung belegt. Anwendungstechnisch zweckmäßig beläuft sich die im Rohrbündelbehälter untergebrachte Anzahl an Kontaktrohren auf wenigstens 5000, vorzugsweise auf wenigstens 10000. Häufig beträgt die Anzahl der im Reaktionsbehälter untergebrachten Kontaktrohre 15000 bis 30000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet (bevorzugt 6 äquidistante Nachbarrohre pro Kontaktrohr), wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. EP-B 468290).

Als Wärmeaustauschmittel eignen sich insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

In der Regel wird bei allen vorstehend erwähnten Konstellationen der Stromführung in den Zweizonenrohrbündelreaktoren der zweiten Reaktionsstufe die Fließgeschwindigkeit innerhalb der beiden erforderlichen Wärmeaustauschmittelkreisläufe so gewählt, dass die Temperatur des Wärmeaustauschmittels von der Eintrittstelle in die Temperaturzone bis zur Austrittsstelle aus der Temperaturzone um 0 bis 15°C ansteigt. D.h., das vorgenannte ΔT kann erfindungsgemäß 1 bis 10°C, oder 2 bis 8°C oder 3 bis 6°C betragen.

Die Eintrittstemperatur des Wärmeaustauschmittels in die Temperaturzone C liegt erfindungsgemäß normalerweise im Bereich von 230 bis 320°C, bevorzugt im Bereich von 250 bis 300°C und besonders bevorzugt im Bereich von 260 bis 280°C. Bei Acroleinbelastungen der Festbettkatalysatorschüttung 2 von 2: 70 NVI.h und ≤ 150 Nl/l·h wird die Eintrittstemperatur des Wärmeaustauschmittels in die Temperaturzone D erfindungsgemäß ebenfalls im Bereich von 230 bis 320°C, gleichzeitig aber normalerweise erfindungsgemäß zweckmäßig ≥ 0°C bis ≤ 20°C oder ≤ 10°C, bzw. ≥ 0°C und ≤ 5°C, oder häufig ≥ 0°C und ≤ 3°C unterhalb der Eintrittstemperatur des in die Temperaturzone C eintretenden Wärmeaustauschmittels liegen. Bei Acroleinbelastung der Festbettkatalysatorschüttung von ≥ 150 Nl/l·h und (in der Regel) ≤ 300 Nl/l·h (bzw. 600 Nl/l·h) wird die Eintrittstemperatur des Wärmeaustauschmittels in die Temperaturzone D erfindungsgemäß ebenfalls im Bereich von 230 bis 320°C, gleichzeitig aber normalerweise erfindungsgemäß zweckmäßig ≥ 0°C bis ≤ 40°C, oder ≥ 5°C und ≤ 35°C, bzw. 30°C, oder ≥ 10°C und ≤ 25°C, bzw. 20°C, oder 15°C oberhalb der Eintrittstemperatur des in die Temperaturzone C eintretenden Wärmeaustauschmittels liegen.

Es sei an dieser Stelle auch noch einmal darauf hingewiesen, dass für eine Durchführung der zweiten Reaktionsstufe des erfindungsgemäßen Verfahrens insbesondere auch der in der DE-AS 2201528 beschriebene Zweizonenrohrbündelreaktortyp verwendet werden kann, der die Möglichkeit beinhaltet, vom heißeren Wärmeaustauschmittel der Temperaturzone D eine Teilmenge an die Temperaturzone C abzuführen, um gegebenenfalls ein Anwärmen eines zu kalten Reaktionsgasausgangsgemisches 2 oder eines kalten Kreisgases zu bewirken. Ferner kann die Rohrbündelcharakteristik, innerhalb einer individuellen Temperaturzone wie in der EP-A 382 098 beschrieben gestaltet werden.

Selbstredend können zur Durchführung des erfindungsgemäßen Verfahrens zwei Zweizonenrohrbündelreaktoren zu einem Vierzonenrohrbündelreaktor verschmolzen werden, wie es in der WO 01/36364 beschrieben ist. Normalerweise befindet sich in diesen Fällen zwischen der Festbettkatalysatorschüttung 1 und der Festbettkatalysatorschüttung 2 eine Inertschüttung. Allerdings kann auf eine solche Zwischeninertschüttung auch verzichtet werden. Die Länge der Reaktionsrohre entspricht im Verschmelzungsfall vielfach der Summe der Längen der nicht verschmolzenen Rohrbündelreaktoren.

Der Propenanteil im Reaktionsgasausgangsgemisch 1 kann beim erfindungsgemäßen Verfahren z.B. bei Werten von 4 bis 15 Vol.-%, häufig bei 5 bis 12 Vol.-% bzw. 5 bis 8 Vol.-% liegen (jeweils bezogen auf das Gesamtvolumen).

Häufig wird man das erfindungsgemäße Verfahren bei einem Propen : Sauerstoff : indifferente Gase (einschließlich Wasserdampf) Volumenverhältnis im Reaktionsgasausgangsgemisch 1 von 1 : (1,0 bis 3,0) : (5 bis 25), vorzugsweise 1 : (1,7 bis 2,3) : (10 bis 15) durchführen. Im Regelfall wird das indifferente (inerte) Gas zu wenigstens 20 % seines Volumens aus molekularem Stickstoff bestehen. Es kann aber auch zu ≥ 30 Vol.-%, oder zu ≥ 40 Vol.-%, oder zu ≥ 50 Vol.-%, oder zu ≥ 60 Vol.-%, oder zu ≥ 70 Vol.-%, oder zur ≥ 80 Vol.-%, oder zu ≥ 90 Vol.-%, oder zu ≥ 95 Vol.-% aus molekularem Stickstoff bestehen (generell sollen in dieser Schrift inerte Verdünnungsgase solche sein ,die sich beim einmaligen Durchgang durch die jeweilige Reaktionsstufe zu weniger als 5 %, bevorzugt zu weniger als 2 % umsetzen; das sind neben molekularem Stickstoff z.B. Gase wie Propan, Ethan, Methan, Pentan, Butan, $CO_2$, CO, Wasserdampf und/oder Edelgase). Natürlich kann das inerte Verdünnungsgas beim erfindungsgemäßen Verfahren auch zu bis zu 50 mol-%, bzw. bis zu 75 mol-% und mehr aus Propan bestehen. Bestandteil des Verdünnungsgases kann auch Kreisgas sein, wie es nach der Abtrennung der Acrylsäure aus dem Produktgasgemisch verbleibt.

Vorgenannte Zusammensetzungsbereiche gelten sowohl in Fällen von Sekundärgaszufuhr als auch in Fällen wo kein Sekundärgas zugeführt wird.

Erfindungsgemäß günstige Reaktionsgasausgangsgemische 1 sind z.B. auch solche, die aus

| 6 bis 15 (bevorzugt 7 bis 11) Vol-% | Propen, |
|---|---|
| 4 bis 20 (bevorzugt 6 bis 12) Vol.-% | Wasser, |
| $\geq 0$ bis 10 (bevorzugt $\geq 0$ bis 5) Vol.-% | von Propen, Wasser Sauerstoff und Stickstoff verschiedenen Bestandteilen, |

soviel molekularem Sauerstoff, dass das molare Verhältnis von enthaltenem molekularem Sauerstoff zu enthaltenem Propen 1,5 bis 2,5 (bevorzugt 1,6 bis 2,2) beträgt, und als Restmenge bis zu 100 Vol.-% Gesamtmenge aus molekularem Stickstoff zusammengesetzt sind,

wie sie die DE-A 10302715 empfiehlt.

Insbesondere bei hohen Propen- bzw. Acroleinbelastungen der Festbettkatalystorschüttung der jeweiligen Reaktionsstufe wird die Mitverwendung von inerten Verdünnungsgasen mit hoher spezifischer Wärme empfohlen.

Der Acroleinanteil im Reaktionsgasausgangsgemisch 2 kann erfindungsgemäß z.B. bei Werten von 3 bis 15 Vol.%, häufig bei 4 bis 10 Vol.-% bzw. 5 bis 8 Vol.-% liegen (jeweils bezogen auf das Gesamtvolumen).

Häufig wird man das erfindungsgemäße Verfahren mit einem im Reaktionsgasausgangsgemisch 2 vorliegenden Acrolein : Sauerstoff : Wasserdampf : Inertgas - Volumenverhältnis (NI) von 1 : (1 bis 3) : (0 bis 20) : (3 bis 30), vorzugsweise von 1 : (1 bis 3) : (0,5 bis 10) : (7 bis 10) ausführen.

Selbstredend kann man das erfindungsgemäße Verfahren aber auch mit einem im Reaktionsgasausgangsgemisch 2 vorliegenden Acrolein : Sauerstoff : Wasserdampf : Sonstige - Volumenverhältnis (NI) von 1 : (0,9 bis 1,3) : (2,5 bis 3,5) : (10 bis 12) ausführen.

An dieser Stelle sei noch festgehalten, dass als Aktivmassen sowohl für die Festbettkatalysatorschüttung 1 als auch für die Festbettkatalysatorschüttung 2 auch die Multimetalloxidmassen der DE-A 10261186 günstig sind.

Erfindungsgemäß günstige Ausgestaltungen eines Zweizonenrohrbündelreaktors für die erste Reaktionsstufe können wie folgt beschaffen sein (die konstruktive Detailgestaltung kann wie in den Gebrauchsmusteranmeldungen 202 19 277.6, 2002 19 278.4 und 202 19 279.2 bzw. in den PCT-Anmeldungen PCT/EP02/14187, PCT/EP02/14188 oder PCT/EPO2/14189 erfolgen):

Kontaktrohre:

| Material der Kontaktrohre: | ferritischer Stahl; |
|---|---|
| Abmessungen der Kontaktrohre: | z.B. 3500 mm Länge; |
| | z.B. 30 mm Außendurchmesser; |
| | z.B. 2 mm Wandstärke; |

Anzahl der Kontaktrohre im Rohrbündel: z.B. 30000, oder 28000, oder 32000, oder 34000; zusätzlich bis zu 10 Thermorohre (wie in EP-A 873 783 und EP-A 12 70 065 beschrieben), die wie die Kontaktrohre beschickt sind (schneckenartig von ganz außen nach innen drehend) z.B. der selben Länge und Wandstärke, aber mit einem Außendurchmesser von z.B. 33,4 mm und einer zentrierten Thermohülse von z.B. 8 mm Außendurchmesser und z.B. 1 mm Wandstärke;

Reaktor (Material wie die Kontaktrohre):

Zylinderförmiger Behälter eines Innendurchmessers von 6000 - 8000 mm;

Reaktorhauben plattiert mit Edelstahl des Typs 1,4541; Plattierungsdicke: einige mm;

ringförmig angeordnetes Rohrbündel, z.B. mit einem freien zentralen Raum;

Durchmesser des zentralen freien Raumes: z.B. 1000 - 2500 mm (z.B. 1200 mm, oder 1400 mm, oder 1600 mm, oder 1800 mm, oder 2000 mm, oder 2200 mm, oder 2400 mm);

normalerweise homogene Kontaktrohrverteilung im Rohrbündel (6 äquidistante Nachbarrohre pro Kontaktrohr), Anordnung in gleichseitigem Dreieck, Kontaktrohrteilung (Abstand der zentrischen Innenachsen von zueinander nächstliegenden Kontaktrohren): 35 - 45 mm, z.B. 36 mm, oder 38 mm, oder 40 mm, oder 42 mm, oder 44 mm;

die Kontaktrohre sind mit ihren Enden in Kontaktrohrböden (oberer Boden und unterer Boden z.B. mit einer Dicke von 100 - 200 mm) abdichtend befestigt und münden am oberen Ende in eine mit dem Behälter verbundene Haube, die einen Zulass für das Reaktionsgasausgangsgemisch 1 aufweist; ein z.B. auf der halben Kontaktrohrlänge befindliches Trennblech einer Dicke von 20 - 100 mm, teilt den Reaktorraum symmetrisch in zwei Temperaturzonen A (obere Zone) und B (untere Zone); jede Temperaturzone wird durch eine Umlenkscheibe in 2 äquidistante Längsabschnitte geteilt;

die Umlenkscheibe weist bevorzugt Ringgeometrie auf; die Kontaktrohre sind mit Vorteil am Trennblech abdichtend befestigt; an den Umlenkscheiben sind sie nicht abdichtend befestigt, so dass die Querströmungsgeschwindigkeit der Salzschmelze innerhalb einer Zone möglichst konstant ist;

jede Zone wird durch eine eigene Salzpumpe mit Salzschmelze als Wärmeträger versorgt; die Zufuhr der Salzschmelze ist z.B. unterhalb der Umlenkscheibe und die Entnahme ist z.B. oberhalb der Umlenkscheibe;

aus beiden Salzschmelzekreisen wird z.B. ein Teilstrom entnommen und z.B. in einem gemeinsamen oder zwei getrennten indirekten Wärmetauschern abgekühlt (Dampferzeugung);

im ersten Fall wird der abgekühlte Salzschmelzestrom aufgeteilt, mit dem jeweiligen Reststrom vereinigt und durch die jeweilige Pumpe in den entsprechenden Ringkanal, der die Salzschmelze über den Behälterumfang verteilt, in den Reaktor gedrückt;

durch im Reaktormantel befindliche Fenster gelangt die Salzschmelze zum Rohrbündel; die Einströmung erfolgt z.B. in radialer Richtung zum Rohrbündel;

die Salzschmelze fließt in jeder Zone der Vorgabe des Umlenkblechs folgend z.B. in der Abfolge

- von außen nach innen,
- von innen nach außen,

um die Kontaktrohre;
durch um den Behälterumfang angebrachte Fenster sammelt sich die Salzschmelze an jedem Zonenende in einem um den Reaktormantel angebrachten Ringkanal, um einschließlich Teilstromkühlung im Kreis gepumpt zu werden;
über jede Temperaturzone wird die Salzschmelze von unten nach oben geführt;
Das Reaktionsgasgemisch verlässt den Reaktor der ersten Stufe mit einer Temperatur wenige Grad höher als die Salzbadeintrittstemperatur des ersten Reaktors. Das Reaktionsgasgemisch wird für die Weiterverarbeitung zweckmäßigerweise in einem separaten Nachkühler, der dem Reaktor der 1. Stufe nachgeschaltet ist, auf 220°C bis 280°C, bevorzugt 240°C bis 260°C abgekühlt.
Der Nachkühler ist in der Regel unterhalb des unteren Rohrbodens angeflanscht und besteht normalerweise aus Rohren mit ferritischem Stahl. In die Rohre des Nachkühlers sind mit Vorteil innen Edelstahl-Blechspiralen, die teil- oder vollgewendelt sein können, zur Verbesserung des Wärmeübergangs eingeführt.

Salzschmelze:

Als Salzschmelze kann ein Gemisch aus 53 Gew.-% Kaliumnitrat, 40 Gew.-% Natriumnitrit und 7 Gew.-% Natriumnitrat verwendet werden; beide Reaktionszonen und der Nachkühler wenden mit Vorteil eine Salzschmelze derselben Zusammensetzung an; die in den Reaktionszonen umgepumpte Salzmenge kann je Zone ca. 10000 $m^3$/h betragen.

Stromführung:

das Reaktionsgasausgangsgemisch 1 strömt zweckmäßig von oben nach unten durch den Erststufenreaktor, während die unterschiedlich temperierten Salzschmelzen der einzelnen Zonen zweckmäßig von unten nach oben gefördert werden;

Kontaktrohr- und Thermorohrbeschickung (von oben nach unten) z.B.:

Abschnitt 1: 50 cm Länge
Steatitringe der Geometrie 7 mm x 7 mm x 4 mm (Außendurchmesser x Länge x Innendurchmesser) als Vorschüttung.

Abschnitt 2: 140 cm Länge
Katalysatorbeschickung mit einem homogenen Gemisch aus 30 Gew.-% an Steatitringen der Geometrie 5 mm x 3 mm x 2 mm (Außendurchmesser x Länge x Innendurchmesser) und 70 Gew.-% Vollkatalysator aus Abschnitt 3.

Abschnitt 3: 160 cm Länge
Katalysatorbeschickung mit ringförmigem (5 mm x 3 mm x 2 mm = Außendurchmesser x Länge x Innendurchmesser) Vollkatalysator gemäß Beispiel 1 der DE-A 10046957 (Stöchiometrie: $[Bi_2W_2O_9$ x 2 $WO_3]_{0,5}$ $[Mo_{12}Co_{5,5}Fe_{2,94}Si_{1,59}K_{0,08}O_x]_1)$.

Erfindungsgemäß günstige Ausgestaltungen eines Zweizonenrohrbündelreaktors für die zweite Reaktionsstufe können wie folgt beschaffen sein:

Alles wie beim Zweizonenrohrbündelreaktor für die erste Reaktionsstufe. Die Dicke des oberen und unteren Kontaktrohrbodens beträgt jedoch häufig 100 - 200 mm, z.B. 110 mm, oder 130 mm, oder 150 mm, oder 170 mm, oder 190 mm.

Der Nachkühler entfällt; statt dessen münden die Kontaktrohre mit ihren unteren Öffnungen in eine am unteren Ende mit dem Behälter verbundene Haube mit Auslass für das Produktgasgemisch; die obere Temperaturzone ist die Zone C und die untere Temperaturzone ist die Temperaturzone D. Zwischen Auslass "Nachkühler" und Einlass "Reaktor für die zweite Reaktionsstufe" besteht zweckmäßig eine Zufuhrmöglichkeit für komprimierte Luft.

Die Kontaktrohr- und Thermorohrbeschickung (von oben nach unten) kann z.B. wie folgt sein:

Abschnitt 1: 20 cm Länge
Steatitringe der Geometrie 7 mm x 7 mm x 4 mm (Außendurchmesser x Länge x Innendurchmesser) als Vorschüttung.

Abschnitt 2: 90 cm Länge
Katalysatorbeschickung mit einem homogenen Gemisch aus 30 Gew.-% an Steatit-Ringen der Geometrie 7 mm x 3 mm x 4 mm (Außendurchmesser x Länge x Innendurchmesser) und 70 Gew.-% Schalenkatalysator aus Abschnitt 4.

Abschnitt 3: 50 cm Länge
Katalysatorbeschickung mit einem homogenen Gemisch aus 20 Gew.-% an Steatit-Ringen der Geometrie 7 mm x 3 mm x 4 mm (Außendurchmesser x Länge x Innendurchmesser) und 80 Gew.-% Schalenkatalysator aus Abschnitt 4.

Abschnitt 4: 190 cm Länge
Katalysatorbeschickung mit ringförmigem (7 mm x 3 mm x 4 mm = Außendurchmesser x Länge x Innendurchmesser) Schalenkatalysator gemäß Herstellungsbeispiel 5 der DE-A 10046928 (Stöchiometrie: $Mo_{12}V_3W_{1,2}Cu_{2,4}O_x)$.

Die Zweitstufen-Kontaktrohr- und Thermorohrbeschickung kann (von oben nach unten) auch so aussehen:

Abschnitt 1: 20 cm Länge
Steatitringe der Geometrie 7 mm x 7 mm x 4 mm (Außendurchmesser x Länge x Innendurchmesser) als Vorschüttung.

Abschnitt 2: 140 cm Länge
Katalysatorbeschickung mit einem homogenen Gemisch aus 25 Gew.-% an Steatit-Ringen der Geometrie 7 mm x 3 mm x 4 mm (Außendurchmesser x Länge x Innendurchmesser) und 75 Gew.-% Schalenkatalysator aus Abschnitt 3.

Abschnitt 3: 190 cm Länge

Katalysatorbeschickung mit ringförmigem (7 mm x 3 mm x 4 mm = Außendurchmesser x Länge x Innendurchmesser) Schalenkatalysator gemäß Herstellungsbeispiel 5 der DE-A 10046928 (Stöchiometrie: $MO_{12}V_3W_{1,2}Cu_{2,4}O_x$).

In den genannten Erststufenbeschickungen kann der Vollkatalysator aus Beispiel 1 der DE-A 10046957 auch ersetzt werden durch:

a) einen Katalysator gemäß Beispiel 1 c aus der EP-A 15565 oder einen gemäß diesem Beispiel herzustellenden Katalysator, der jedoch die Aktivmasse $Mo_{12}Ni_{6,5}Zn_2Fe_2Bi_1P_{0,0065}K_{0,06}O_x \cdot 10\ SiO_2$ aufweist;

b) Beispiel Nr. 3 aus der DE-A 19855913 als Hohlzylindervollkatalysator der Geometrie 5 mm x 3 mm x 2 mm bzw. 5 mm x 2 mm x 2 mm;

c) Multimetalloxid II - Vollkatalysator gemäß Beispie 1 der DE-A 19746210;

d) einen der Schalenkatalysatoren 1, 2 und 3 aus der DE-A 10063162, jedoch bei gleicher Schalendicke auf Trägerringe der Geometrie 5 mm x 3 mm x 1,5 mm bzw. 7 mm x 3 mm x 1,5 mm aufgebracht.

In allen vorgenannten Zweitstufenbeschickungen kann der Schalenkatalysator gemäß Herstellungsbeispiel 5 der DE-A 10046928 ersetzt werden durch:

a) Schalenkatalysator S1 oder S7 aus der DE-A 4442346 mit einem Aktivmassenanteil von 27 Gew.-% und einer Schalendicke von 230 $\mu$m;

b) einem Schalenkatalysator gemäß den Beispielen 1 bis 5 aus der DE-A 19815281, jedoch auf Trägerringe der Geometrie 7 mm x 3 mm x 4 mm mit einem Aktivmassenanteil von 20 Gew.-% aufgebracht;

c) Schalenkatalysator mit zweiphasiger Aktivmasse der Stöchiometrie $(Mo_{10,4}V_3W_{1,2}O_x)$ $(CuMo_{0,5}W_{0,5}O_4)_{1,6}$, hergestellt gemäß DE-A 19736105 und mit einem Aktivmassenanteil von 20 Gew.-% auf den vorgenannten 7 mm x 3 mm x 4 mm Träger aufgebracht.

Im Übrigen werden die Festbettkatalysatorschüttung 1 und die Festbettkatalysatorschüttung 2 erfindungsgemäß zweckmäßig so gewählt (z.B. durch Verdünnung mit z.B. Inertmaterial), dass der Temperaturunterschied zwischen dem Heißpunktmaximum des Reaktionsgasgemisches in den einzelnen Reaktionszonen und der jeweiligen Temperatur der Reaktionszone in der Regel 80°C nicht überschreitet. Meist beträgt dieser Temperaturunterschied ≤ 70°C, häufig liegt er bei 20 bis 70°C, vorzugsweise ist dieser Temperaturunterschied gering. Außerdem werden die Festbettkatalysatorschüttungen 1 und 2 aus Sicherheitsgründen in dem Fachmann an sich bekannter Weise so gewählt (z.B. durch Verdünnung mit z.B. Inertmaterial), dass die "peak-to-salt-temperature sensitivity" gemäß Definition in der EP-A 1106598 ≤ 9°C, oder ≤ 7°C, oder ≤ 5°C, oder ≤ 3°C beträgt.

Nachkühler und Reaktor für die zweite Stufe sind durch ein Verbindungsrohr verbunden, dessen Länge weniger als 25 m beträgt.

In den nachfolgenden Beispielen und Vergleichsbeispielen sowie in der vorstehenden Reaktoranordnung können in der zweiten Reaktionsstufe die ringförmigen Verdünnungsformkörper und die ringförmigen Katalysatorformkörper auch durch kugelförmige Verdünnungsformkörper und kugelförmige Katalysatorformkörper (jeweils mit Radius 2 bis 5 mm und mit einem Aktivmassenanteil von 10 bis 30 Gew.-%, häufig 10 bis 20 Gew.-%) ersetzt werden.

Beispiele

a) Versuchsanordnung

Erste Reaktionsstufe:

Ein Reaktionsrohr (V2A Stahl; 30 mm Außendurchmesser, 2 mm Wandstärke, 26 mm Innendurchmesser, Länge: 350 cm, sowie ein in der Reaktionsrohrmitte zentriertes Thermorohr (4 mm Außendurchmesser) zur Aufnahme eines Thermoelements mit dem die Temperatur im Reaktionsrohr auf seiner gesamten Länge ermittelt werden kann) wird von oben nach unten wie folgt beschickt:

Abschnitt 1: 50 cm Länge
Steatitringe der Geometrie 7 mm x 7 mm x 4 mm (Außendurchmesser x Länge x Innendurchmesser) als Vorschüttung.

Abschnitt 2: 140 cm Länge
Katalysatorbeschickung mit einem homogenen Gemisch aus 30 Gew.-% an Steatitringen der Geometrie 5 mm x 3 mm x 2 mm (Außendurchmesser x Länge x Innendurchmesser) und 70 Gew.-% Vollkatalysator aus Abschnitt 3.

Abschnitt 3: 160 cm Länge
Katalysatorbeschickung mit ringförmigem (5 mm x 3 mm x 2 mm = Außendurchmesser x Länge x Innendurchmesser) Vollkatalysator gemäß Beispiel 1 der DE-A 10046957 (Stöchiometrie: $[Bi_2W_2O_9$ x $2WO_3]_{0,5}$ $[Mo_{12}Co_{5,5}Fe_{2,94}Si_{1,59}K_{0,08}O_x]_1$).

Von oben nach unten werden die ersten 175 cm mittels eines im Gegenstrom gepumpten Salzbades A thermostatisiert. Die zweiten 175 cm werden mittels eines im Gegenstrom gepumpten Salzbades B thermostatisiert.

Zweite Reaktionsstufe:

Ein Reaktionsrohr (V2A Stahl; 30 mm Außendurchmesser, 2 mm Wandstärke, 26 mm Innendurchmesser, Länge: 350 cm, sowie ein in der Reaktionsrohrmitte zentriertes Thermorohr (4 mm Außendurchmesser) zur Aufnahme eines Thermoelements mit dem die Temperatur im Reaktionsrohr auf seiner gesamten Länge ermittelt werden kann) wird von oben nach unten wie folgt beschickt:

Abschnitt 1: 20 cm Länge
Steatitringe der Geometrie 7 mm x 7 mm x 4 mm (Außendurchmesser x Länge x Innendurchmesser) als Vorschüttung.

Abschnitt 2: 90 cm Länge
Katalysatorbeschickung mit einem homogenen Gemisch aus 30 Gew.-% an Steatit-Ringen der Geometrie 7 mm x 3 mm x 4 mm (Außendurchmesser x Länge x Innendurchmesser) und 70 Gew.-% Schalenkatalysator aus Abschnitt 4.

Abschnitt 3: 50 cm Länge
Katalysatorbeschickung mit einem homogenen Gemisch aus 20 Gew.-% an Steatit-Ringen der Geometrie 7 mm x 3 mm x 4 mm (Außendurchmesser x Länge x Innendurchmesser) und 80 Gew.-% Schalenkatalysator aus Abschnitt 4.

Abschnitt 4: 190 cm Länge
Katalysatorbeschickung mit ringförmigem (7 mm x 3 mm x 4 mm = Außendurchmesser x Länge x Innendurchmesser) Schalenkatalysator gemäß Herstellungsbeispiel 5 der DE-A 10046928 (Stöchiometrie: $Mo_{12}V_3W_{1,2}Cu_{2,4}O_x$).

Von oben nach untern werden die ersten 175 cm mittels eines im Gegenstrom gepumpten Salzbades C thermostatisiert. Die zweiten 175 cm werden mittels eines im Gegenstrom gepumpten Salzbades D thermostatisiert.

Gasphasenoxidation:

Die vorstehend beschriebene erste Reaktionsstufe wird mit einem Reaktionsgasausgangsgemisch 1 der nachfolgenden Zusammensetzung kontinuierlich beschickt, wobei die Belastung und die Thermostatisierung des ersten Reaktionsrohres variiert werden:

6 bis 6,5 Vol.-% Propen,
3 bis 3,5 Vol.-% $H_2O$,
0,3 bis 0,5 Vol.-% CO,
0,8 bis 1,2 Vol.-% $CO_2$,
0,01 bis 0,04 Vol.-% Acrolein,
10,4 bis 10,7 Vol.-% $O_2$ und

als Restmenge ad 100 % molekularer Stickstoff.

Dem Produktgasgemisch der ersten Reaktionsstufe wird am Ausgang des ersten Reaktionsrohres eine kleine Probe für eine gaschromatographische Analyse entnommen. In übrigen wird das Produktgasgemisch unter Zudüsen von eine Temperatur von 25°C aufweisender Luft unmittelbar in die nachfolgende Acroleinoxidationsstufe (zu Acrylsäure) geführt (Reaktionsstufe 2).

Vom Produktgasgemisch der Acroleinoxidationsstufe wird ebenfalls eine kleine Probe für eine gaschromatographische Analyse entnommen. Im übrigen wird die Acrylsäure vom Produktgasgemisch der zweiten Reaktionsstufe in an sich bekannter Weise abgetrennt und ein Teil des verbleibenden Restgases zur Beschickung der Propenoxidationsstufe wiederverwendet (als sogenanntes Kreisgas), was den Acroleingehalt des vorgenannten Beschickungsgases und die geringe Varianz der Feedzusammensetzung erklärt.

In beiden Reaktionsstufen durchströmt das Reaktionsgasgemisch die beiden Kontaktrohre von oben nach unten.

Der Druck am Eingang der ersten Reaktionsstufe variiert in Abhängigkeit von der gewählten Propenbelastung zwischen 2,3 und 3,1 bar. Am Ende der Reaktionszonen A, C befindet sich ebenfalls eine Analysenstelle. Der Druck am Eingang der zweiten Reaktionsstufe variiert in Abhängigkeit von der Acroleinbelastung zwischen 1,6 und 2,1 bar.

Die Ergebnisse in Abhängigkeit von Belastungen und Salzbadtemperaturen sowie der Luftzugabe nach der ersten Reaktionsstufe zeigt die nachfolgende Tabelle 1 (der Buchstabe B in Klammern bedeutet Beispiel).

| | |
|---|---|
| $T_A, T_B, T_C, T_D$ | stehen für die Temperaturen der umgepumpten Salzbäder in den Reaktionszonen A, B, C und D in °C. |
| $U^P_A$ | ist der Propenumsatz am Ende der Reaktionszone A in mol-%. |
| $U^P_B$ | ist der Propenumsatz am Ende der Reaktionszone B in mol-%. |
| $S^{WP}$ | ist die Selektivität der Acroleinbildung sowie der Acrylsäurenebenproduktbildung zusammengenommen nach der ersten Reaktionsstufe und bezogen auf umgesetztes Propen in mol-%. |
| $U^{AC}_C$ | ist der Acroleinumsatz am Ende der Reaktionszone C in mol%. |
| $U^{AC}_D$ | ist der Acroleinumsatz am Ende der Reaktionszone D in mol-%. |
| $U^P_D$ | ist der Propenumsatz am Ende der Reaktionszone D in mol-% (bezogen auf die Eingangsmenge an Propen in die Reaktionszone A). |
| $S^{AA}$ | ist die Selektivität der Acrylsäurebildung nach der zweiten Reaktionsstufe und bezogen auf umgesetztes Propen in mol-%. |
| V | ist das molare Verhältnis von molekularem Sauerstoff : Acrolein im Reaktionsgasausgangsgemisch 2. |
| M | ist die Luftzugabe nach der ersten Reaktionsstufe in Nl/h. |
| $T^{maxA}, T^{maxB}, T^{maxC}, T^{maxD}$ | stehen für die höchste Temperatur des Reaktionsgasgemisches innerhalb der Reaktionszonen A, B, C und D in °C. |

b) Ergebnisse

Tabelle 1

| Propenbelastung (Nl/l·h) | $T_A$ | $T_B$ | $T^{maxA}$ | $T^{maxB}$ | $U^P_A$ | $U^P_B$ | $S^{WP}$ | M | V |
|---|---|---|---|---|---|---|---|---|---|
| 130 (B) | 319 | 319 | 384 | 351 | 66,7 | 95,1 | 97,7 | 404 | 1,40 |
| 130 (B) | 327 | 313 | 400 | 330 | 70,3 | 94,8 | 98,3 | 404 | 1,38 |

(fortgesetzt)

| Propenbelastung (NI/l·h) | $T_A$ | $T_B$ | $T^{maxA}$ | $T^{maxB}$ | $UP_A$ | $UP_B$ | $S^{WP}$ | M | V |
|---|---|---|---|---|---|---|---|---|---|
| 185 (V) | 322 | 336 | 380 | 368 | 64,5 | 94,9 | 98,0 | 574 | 1,39 |

Tabelle 1 Fortsetzung

| Acroleinbelastung (NI/l·h) | $T_C$ | $T_D$ | $T^{maxC}$ | $T^{maxD}$ | $U^{AC}_C$ | $U^{AC}_D$ | $UP_D$ | $S^{AA}$ |
|---|---|---|---|---|---|---|---|---|
| 106 (B) | 260 | 260 | 302 | 276 | 80,7 | 99,3 | 95,1 | 95,4 |
| 108 (B) | 262 | 259 | 312 | 275 | 84,8 | 99,3 | 94,8 | 95,8 |
| 152 (V) | 263 | 269 | 303 | 287 | 78,8 | 99,3 | 94,9 | 95,8 |

Vergleichsbeispiele

Alles wird wie in den Beispielen durchgeführt. Die Länge der Beschickungsabschnitte der Festbettkatalysatorbeschickungen 1 und 2 wird jedoch wie folgt geändert:

Erste Reaktionsstufe:

|  | Abschnitt 2: | 125 cm anstelle 140 cm. |
|---|---|---|
|  | Abschnitt 3: | 175 cm anstelle 160 cm. |

Zweite Reaktionsstufe:

|  | Abschnitt 3: | 65 cm anstelle 50 cm. |
|---|---|---|
|  | Abschnitt 4: | 175 cm anstelle 190 cm. |

Die diesen Bedingungen entsprechenden Ergebnisse zeigt die Tabelle 2.

Tabelle 2

| Propenbelastung (NI/l · h) | $T_A$ | $T_B$ | $T^{maxA}$ | $T^{maxB}$ | $UP_A$ | $UP_B$ | $S^{WP}$ | M | V |
|---|---|---|---|---|---|---|---|---|---|
| 130 (V) | 319 | 315 | 384 | 360 | 66,4 | 94,9 | 97,4 | 404 | 1,40 |
| 130 (V) | 327 | 310 | 400 | 339 | 70,2 | 95,0 | 98,0 | 404 | 1,38 |
| 185 (V) | 322 | 331 | 380 | 377 | 64,7 | 94,8 | 97,7 | 574 | 1,39 |

Tabelle 2 Fortsetzung

| Acroleinbelastung (NI/l · h) | $T_C$ | $T_D$ | $T^{maxC}$ | $T^{maxD}$ | $U^{AC}_C$ | $U^{AC}_D$ | $UP_D$ | $S^{AA}$ |
|---|---|---|---|---|---|---|---|---|
| 103 (V) | 265 | 260 | 323 | 276 | 80,8 | 99,3 | 94,9 | 95,2 |
| 106 (V) | 268 | 259 | 332 | 275 | 84,9 | 99,2 | 95,0 | 95,0 |
| 150 (V) | 267 | 269 | 326 | 287 | 78,6 | 99,3 | 94,8 | 95,3 |

Im Vergleich mit den Ergebnissen in Tabelle 1 werden bei vergleichbaren Umsatzwerten geringere $S^{AA}$ erzielt.

**Patentansprüche**

1. Verfahren der heterogen katalysierten partiellen Gasphasenoxidation von Propen zu Acrylsäure, bei dem man ein Propen, molekularen Sauerstoff und wenigstens ein Inertgas enthaltendes Reaktionsgasausgangsgemisch 1 in einer ersten Reaktionsstufe mit der Maßgabe über eine Festbettkatalysatorschüttung 1, deren Aktivmasse wenigstens ein die Elemente Mo, Fe und Bi enthaltendes Multimetalloxid ist, führt, so dass

- die Festbettkatalysatorschüttung 1 in zwei räumlich aufeinanderfolgenden Temperaturzonen A, B angeordnet ist,
- sowohl die Temperatur der Temperaturzone A als auch die Temperatur der Temperaturzone B eine Temperatur im Bereich von 290 bis 380°C ist,
- die Festbettkatalysatorschüttung 1 aus wenigstens zwei räumlich aufeinander folgenden Festbettkatalysatorschüttungszonen besteht, wobei die volumenspezifische Aktivität innerhalb einer Festbettkatalysatorschüttungszone im wesentlichen konstant ist und in Strömungsrichtung des Reaktionsgasgemisches 1 beim Übergang von einer Festbettkatalysatorschüttungszone in eine andere Festbettkatalysatorschüttungszone sprunghaft zunimmt,
- sich die Temperaturzone A bis zu einem Umsatz des Propens von 40 bis 80 mol-% erstreckt,
- bei einmaligem Durchgang des Reaktionsgasausgangsgemisches 1 durch die gesamte Festbettkatalysatorschüttung 1 der Propenumsatz $\geq$ 90 mol-% und die Selektivität der Acroleinbildung sowie der Acrylsäurenebenproduktbildung zusammengenommen und bezogen auf umgesetztes Propen $\geq$ 90 mol-% beträgt,
- die zeitliche Abfolge, in der das Reaktionsgasgemisch 1 die Temperaturzonen A, B durchströmt, der alphabetischen Abfolge der Temperaturzonen A, B entspricht,
- die Belastung der Festbettkatalysatorschüttung 1 mit dem im Reaktionsgasausgangsgemisch 1 enthaltenen Propen $\geq$ 90 Nl Propen/l Festbettkatalysatorschüttung 1 · h beträgt, und
- die Differenz $T^{maxA}$ - $T^{maxB}$, gebildet aus der höchsten Temperatur $T^{maxA}$, die das Reaktionsgasgemisch 1 innerhalb der Temperaturzone A aufweist, und der höchsten Temperatur $T^{maxB}$, die das Reaktionsgasgemisch 1 innerhalb der Temperaturzone B aufweist, $\geq$ 0°C beträgt,

die Temperatur des die erste Reaktionsstufe verlassenden Produktgasgemisches durch Kühlung gegebenenfalls verringert und dem Produktgasgemisch gegebenenfalls molekularen Sauerstoff und/oder Inertgas zugibt, und danach das Produktgasgemisch als Acrolein, molekularen Sauerstoff und wenigstens ein Inertgas enthaltendes Reaktionsgasausgangsgemisch 2, das den molekularen Sauerstoff und das Acrolein in einem molaren Verhältnis $O_2$:$C_3H_4O \geq$ 0,5 enthält, in einer zweiten Reaktionsstufe mit der Maßgabe über eine Festlaettkatalysatorschüttung 2, deren Aktivmasse wenigstens ein die Elemente Mo und V enthaltendes Multimetalloxid ist, führt, dass

- die Festbettkatalysatorschüttung 2 in zwei räumlich aufeinanderfolgenden Temperaturzonen C, D angeordnet ist,
- sowohl die Temperatur der Temperaturzone C als auch die Temperatur der Temperaturzone D eine Temperatur im Bereich von 230 bis 320°C ist,
- die Festbettkatalysatorschüttung 2 aus wenigstens zwei räumlich aufeinander folgenden Festbettkatalysatorschüttungszonen besteht, wobei die volumenspezifische Aktivität innerhalb einer Festbettkatalysatorschüttungszone im wesentlichen konstant ist und in Strömungsrichtung des Reaktionsgasgemisches 2 beim Übergang von einer Festbettkatalysatorschüttungszone in eine andere Festbettkatalysatorschüttungszone sprunghaft zunimmt,
- sich die Temperaturzone C bis zu einem Umsatz des Acroleins von 45 bis 85 mol-% erstreckt,
- bei einmaligem Durchgang des Reaktionsgasausgangsgemisches 2 durch die gesamte Festbettkatalysatorschüttung 2 der Acroleinumsatz $\geq$ 90 mol-% und die Selektivität der Acrylsäurebildung, bezogen auf über beide Reaktionsstufen umgesetztes Propen, $\geq$ 80 mol-% beträgt,
- die zeitliche Abfolge, in der das Reaktionsgasgemisch die Temperaturzonen C, D durchströmt, der alphabetischen Abfolge der Temperaturzonen C, D entspricht,
- die Belastung der Festbettkatalysatorschüttung 2 mit dem im Reaktionsgasausgangsgemisch 2 enthaltenen Acrolein $\geq$ 70 Nl Acrolein/l Festbettkatalysatorschüttung 2 · h beträgt, und
- die Differenz $T^{maxC}$ - $T^{maxD}$, gebildet aus der höchsten Temperatur $T^{maxC}$, die das Reaktionsgasgemisch innerhalb der Temperaturzone C aufweist, und der höchsten Temperatur $T^{maxD}$, die das Reaktionsgasgemisch innerhalb der Temperaturzone D aufweist, $\geq$ 0°C beträgt,

**dadurch gekennzeichnet, dass** weder der Übergang von der Temperaturzone A in die Temperaturzone B in der Festbettkatalysatorschüttung 1, noch der Übergang von der Temperaturzone C in die Temperaturzone D in der Festbettkatalysatorschüttung 2 mit einem Übergang von einer Festbettkatalysatorschüttungszone in eine andere Festbettkatalysatorschüttungszone zusammenfällt.

2. Verfahren nach Anspruch 1, **dadurch gekennzeichnet, dass** $T^{maxA}$ - $T^{maxB}$ ≥ 3°C und ≤ 70°C beträgt.

3. Verfahren nach Anspruch 1 oder 2, **dadurch gekennzeichnet, dass** $T^{maxC}$ - $T^{maxD}$ ≥ 3°C und ≤ 60°C beträgt.

4. Verfahren nach einem der Ansprüche 1 bis 3, **dadurch gekennzeichnet, dass** die Propenbelastung der Festbettkatalysatorschüttung 1 ≥ 90 Nl/l · h und < 160 Nl/l · h beträgt.

5. Verfahren nach einem der Ansprüche 1 bis 3, **dadurch gekennzeichnet, dass** die Propenbelastung der Festbettkatalysatorschüttung 1 ≥ 160 Nl/l · h und ≤ 300 Nl/l · h beträgt.

6. Verfahren nach einem der Ansprüche 1 bis 5, **dadurch gekennzeichnet, dass** die chemische Zusammensetzung der verwendeten Aktivmasse über die gesamte Festbettkatalysatorschüttung 1 unverändert ist.

7. Verfahren nach einem der Ansprüche 1 bis 6, **dadurch gekennzeichnet, dass** die gesamte Festbettkatalysatorschüttung 1 nicht mehr als vier Festbettkatalysatorschüttungszonen umfasst.

8. Verfahren nach einem der Ansprüche 1 bis 7, **dadurch gekennzeichnet, dass** bei einheitlicher Aktivmasse über die gesamte Festbettkatalysatorschüttung 1 beim Übergang von einer Festbettkatalysatorschüttungszone in eine andere Festbettkatalysatorschüttungszone in der Festbettkatalysatorschüttung 1 in Strömungsrichtung des Reaktionsgasgemisches 1 die volumenspezifische Aktivmasse um wenigstens 5 Gew.-% zunimmt.

9. Verfahren nach einem der Ansprüche 1 bis 8, **dadurch gekennzeichnet, dass** bei einheitlicher Aktivmasse über die gesamte Festbettkatalysatorschüttung 1 der Unterschied in der volumenspezifischen Aktivmasse derjenigen Festbettkatalysatorschüttungszone in der Festbettkatalysatorschüttung 1 mit der geringsten volumenspezifischen Aktivität und derjenigen Festbettkatalysatorschüttungszone in der Festbettkatalysatorschüttung 1 mit der höchsten volumenspezifischen Aktivität nicht mehr als 40 Gew.-% beträgt.

10. Verfahren nach einem der Ansprüche 1 bis 9, **dadurch gekennzeichnet, dass** die in Strömungsrichtung des Reaktionsgasgemisches 1 letzte Festbettkatalysatorschüttungszone der Festbettkatalysatorschüttung 1 unverdünnt ist und nur aus Katalysatorformkörpern besteht.

11. Verfahren nach einem der Ansprüche 1 bis 10, **dadurch gekennzeichnet, dass** die Festbettkatalysatorschüttungszone mit der höchsten volumenspezifischen Aktivität innerhalb der Festbettkatalysatorschüttung 1 nicht bis in die Temperaturzone A hineinragt.

12. Verfahren nach einem der Ansprüche 1 bis 11, **dadurch gekennzeichnet, dass** sich in der Festbettkatalysatorschüttung 1 im Bereich $X^1 \pm L^1 \dfrac{4}{100}$ kein Übergang von einer Festbettkatalysatorschüttungszone in eine andere Festbettkatalysatorschüttungszone befindet, wobei $L^1$ die Länge der Festbettkatalysatorschüttung 1 und $X^1$ die Stelle innerhalb der Festbettkatalysatorschüttung 1 ist, wo der Übergang von der Temperaturzone A in die Temperaturzone B erfolgt.

13. Verfahren nach einem der Ansprüche 1 bis 12, **dadurch gekennzeichnet, dass** die chemische Zusammensetzung der verwendeten Aktivmasse über die gesamte Festbettkatalysatorschüttung 2 unverändert ist.

14. Verfahren nach einem der Ansprüche 1 bis 13, **dadurch gekennzeichnet, dass** die gesamte Festbettkatalysatorschüttung 2 nicht mehr als vier Festbettkatalysatorschüttungszonen umfasst.

15. Verfahren nach einem der Ansprüche 1 bis 14, **dadurch gekennzeichnet, dass** bei einheitlicher Aktivmasse über die gesamte Festbettkatalysatorschüttung 2 beim Übergang von einer Festbettkatalysatorschüttungszone in eine andere Festbettkatalysatorschüttungszone innerhalb der Festbettkatalysatorschüttung 2 in Strömungsrichtung des Reaktionsgasgemisches die volumenspezifische Aktivmasse um wenigstens 5 Gew.-% zunimmt.

**16.** Verfahren nach einem der Ansprüche 1 bis 15, **dadurch gekennzeichnet, dass** bei einheitlicher Aktivmasse über die gesamte Festbettkatalysatorschüttung 2 der Unterschied in der volumenspezifischen Aktivmasse derjenigen Festbettkatalysatorschüttungszone innerhalb der Festbettkatalysatorschüttung 2 mit der geringsten volumenspezifischen Aktivität und derjenigen Festbettkatalysatorschüttungszone mit der höchsten volumenspezifischen Aktivität innerhalb der Festbettkatalysatorschüttung 2 nicht mehr als 40 Gew.-% beträgt.

**17.** Verfahren nach einem der Ansprüche 1 bis 16, **dadurch gekennzeichnet, dass** die in Strömungsrichtung des Reaktionsgasgemisches letzte Festbettkatalysatorschüttungszone unverdünnt ist und nur aus Katalysatorformkörpern besteht.

**18.** Verfahren nach einem der Ansprüche 1 bis 17, **dadurch gekennzeichnet, dass** die Festbettkatalysatorschüttungszone mit der höchsten volumenspezifischen Aktivität bis in die Temperaturzone C hineinragt.

**19.** Verfahren nach einem der Ansprüche 1 bis 18, **dadurch gekennzeichnet, dass** sich in der Festbettkatalysatorschüttung 2 im Bereich $X^2 \pm L^2 \dfrac{4}{100}$ kein Übergang von einer Festbettkatalysatorschüttungszone in eine andere Festbettkatalysatorschüttungszone befindet, wobei $L^2$ die Länge der Festbettkatalysatorschüttung 2 und $X^2$ die Stelle innerhalb der Festbettkatalysatorschüttung ist, wo der Übergang von der Temperaturzone C in die Temperaturzone D erfolgt.

**Claims**

**1.** A process for partially oxidizing propene to acrylic acid in the gas phase under heterogeneous catalysis by conducting a starting reaction gas mixture 1 comprising propene, molecular oxygen and at least one inert gas in a first reaction stage over a fixed catalyst bed 1 whose active composition is at least one multimetal oxide comprising the elements Mo, Fe and Bi, in such a way that

- the fixed catalyst bed 1 is arranged in two spatially successive temperature zones A, B,
- both the temperature of temperature zone A and the temperature of temperature zone B are a temperature in the range from 290 to 380°C,
- the fixed catalyst bed 1 consists of at least two spatially successive fixed catalyst bed zones, and the volume-specific activity within one fixed catalyst bed zone is substantially constant and increases sharply in the flow direction of the reaction gas mixture 1 at the transition from one fixed catalyst bed zone to another fixed catalyst bed zone,
- the temperature zone A extends up to a conversion of the propene of from 40 to 80 mol%,
- on single pass of the starting reaction gas mixture 1 through the entire fixed catalyst bed 1, the propene conversion is $\geq 90$ mol% and the selectivity of acrolein formation and also of acrylic acid by-production taken together and based on converted propene are $\geq 90$ mol%,
- the sequence in time in which the reaction gas mixture 1 flows through the temperature zones A, B corresponds to the alphabetic sequence of the temperature zones A, B,
- the hourly space velocity of the propene contained in the starting reaction gas mixture 1 on the fixed catalyst bed 1 is $\geq 90$ l (STP) of propene/l of fixed bed catalyst 1·h, and
- the difference $T^{maxA} - T^{maxB}$, formed from the highest temperature $T^{maxA}$ which the reaction gas mixture 1 has within temperature zone A, and the highest temperature $T^{maxB}$ which the reaction gas mixture 1 has within temperature zone B is $\geq 0$°C,

optionally reducing the temperature of the product gas mixture leaving the first reaction stage by cooling and optionally adding molecular oxygen and/or inert gas to the product gas mixture, and afterward conducting the product gas mixture as a starting reaction gas mixture 2 which comprises acrolein, molecular oxygen and at least one inert gas, and contains the molecular oxygen and the acrolein in a molar $O_2:C_3H_4O$ ratio of $\geq 0.5$ in a second reaction stage over a fixed catalyst bed 2 whose active composition is at least one multimetal oxide comprising the elements Mo and V, in such a way that

- the fixed catalyst bed 2 is arranged in two spatially successive temperature zones C, D,
- both the temperature of temperature zone C and the temperature of temperature zone D are a temperature in the range from 230 to 320°C,
- the fixed catalyst bed 2 consists of at least two spatially successive fixed catalyst bed zones, and the volume-specific activity within one fixed catalyst bed zone is substantially constant and increases sharply in the flow direction of the reaction gas mixture 2 at the transition from one fixed catalyst bed zone to another fixed catalyst bed zone,
- the temperature zone C extends up to a conversion of the acrolein of from 45 to 85 mol%,
- on single pass of the starting reaction gas mixture 2 through the entire fixed catalyst bed 2, the acrolein conversion is ≥ 90 mol% and the selectivity of acrylic acid formation, based on propene converted over both reaction stages, is ≥ 80 mol%,
- the sequence in time in which the reaction gas mixture flows through the temperature zones C, D corresponds to the alphabetic sequence of the temperature zones C, D, .
- the hourly space velocity of the acrolein contained in the starting reaction gas mixture 2 on the fixed catalyst bed 2 is ≥ 70 l (STP) of acrolein/l of fixed bed catalyst 2·h, and
- the difference $T^{maxC}$ - $T^{maxD}$, formed from the highest temperature $T^{maxC}$ which the reaction gas mixture has within temperature zone C, and the highest temperature $T^{maxD}$ which the reaction gas mixture has within temperature zone D is ≥ 0°C,

wherein neither the transition from temperature zone A to temperature zone B in fixed catalyst bed 1 nor the transition from temperature zone C to temperature zone D in fixed catalyst bed 2 coincides with a transition from one fixed catalyst bed zone to another fixed catalyst bed zone.

2. A process as claimed in claim 1, wherein $T^{maxA}$ - $T^{maxB}$ is ≥ 3°C and ≤ 70°C.

3. A process as claimed in claim 1 or 2, wherein $T^{maxC}$ - $T^{maxD}$ is ≥ 3°C and ≤ 60°C.

4. A process as claimed in any of claims 1 to 3, wherein the propene hourly space velocity on the fixed catalyst bed 1 is ≥ 90 l (STP)/l·h and < 160 l (STP)/l·h.

5. A process as claimed in any of claims 1 to 3, wherein the propene hourly space velocity on the fixed catalyst bed 1 is ≥ 160 l (STP)/l·h and ≤ 300 l (STP)/l·h.

6. A process as claimed in any of claims 1 to 5, wherein the chemical composition of the active composition used is unchanged over the entire fixed catalyst bed 1.

7. A process as claimed in any of claims 1 to 6, wherein the entire fixed catalyst bed 1 comprises not more than 4 fixed catalyst bed zones.

8. A process as claimed in any of claims 1 to 7, wherein, when the active composition is uniform over the entire fixed catalyst bed 1, the volume-specific active composition in the fixed catalyst bed 1 in the flow direction of the reaction gas mixture 1 increases by at least 5% by weight at the transition from one fixed catalyst bed zone to another fixed catalyst bed zone.

9. A process as claimed in any of claims 1 to 8, wherein, when the active composition is uniform over the entire fixed catalyst bed 1, the difference in the volume-specific active composition between the fixed catalyst bed zone in the fixed catalyst bed 1 having the lowest volume-specific activity and the fixed catalyst bed zone in the fixed catalyst bed 1 having the highest volume-specific activity is not more than 40% by weight.

10. A process as claimed in any of claims 1 to 9, wherein the last fixed catalyst bed zone of the fixed catalyst bed 1 in the flow direction of the reaction gas mixture 1 is undiluted and consists only of shaped catalyst bodies.

11. A process as claimed in any of claims 1 to 10, wherein the fixed catalyst bed zone having the highest volume-specific activity within the fixed catalyst bed 1 does not extend into temperature zone A.

12. A process as claimed in any of claims 1 to 11, wherein there is no transition from one fixed catalyst bed zone to another fixed catalyst bed zone in the fixed catalyst bed 1 within the range of $X^1 \pm L^1 \dfrac{4}{100}$ where $L^1$ is the length

of the fixed catalyst bed 1 and $X^1$ is the point within the fixed catalyst bed 1 of transition from temperature zone A to temperature zone B.

13. A process as claimed in any of claims 1 to 12, wherein the chemical composition of the active composition used is unchanged over the entire fixed catalyst bed 2.

14. A process as claimed in any of claims 1 to 13, wherein the entire fixed catalyst bed 2 comprises not more than 4 fixed catalyst bed zones.

15. A process as claimed in any of claims 1 to 14, wherein, when the active composition is uniform over the entire fixed catalyst bed 2, the volume-specific active composition within the fixed catalyst bed 2 in the flow direction of the reaction gas mixture increases by at least 5% by weight at the transition from one fixed catalyst bed zone to another fixed catalyst bed zone.

16. A process as claimed in any of claims 1 to 15, wherein, when the active composition is uniform over the entire fixed catalyst bed 2, the difference in the volume-specific active composition between the fixed catalyst bed zone within the fixed catalyst bed 2 having the lowest volume-specific activity and the fixed catalyst bed zone within the fixed catalyst bed 2 having the highest volume-specific activity is not more than 40% by weight.

17. A process as claimed in any of claims 1 to 16, wherein the last fixed catalyst bed zone in the flow direction of the reaction gas mixture is undiluted and consists only of shaped catalyst bodies.

18. A process as claimed in any of claims 1 to 17, wherein the fixed catalyst bed zone having the highest volume-specific activity does not extend into temperature zone C.

19. A process as claimed in any of claims 1 to 18, wherein there is no transition from one fixed catalyst bed zone to another fixed catalyst bed zone in the fixed catalyst bed 2 within the range of $X^2 \pm L^2 \dfrac{4}{100}$ where $L^2$ is the length of the fixed catalyst bed 2 and $X^2$ is the point within the fixed catalyst bed of transition from temperature zone C to temperature zone D.

## Revendications

1. Procédé d'oxydation partielle en phase gazeuse à catalyse hétérogène de propène en acide acrylique, dans lequel on achemine un mélange initial de gaz réactionnel 1 contenant du propène, de l'oxygène moléculaire et au moins un gaz inerte dans une première étape réactionnelle sur un catalyseur en vrac à lit fixe 1 dont la masse active est au moins un oxyde de plusieurs métaux contenant les éléments Mo, Fe et Bi de sorte :

   - que le catalyseur en vrac à lit fixe 1 soit agencé dans deux zones de température A, B se succédant spatialement,
   - que la température de la zone de température A ainsi que la température de la zone de température B se situent dans la plage de 290 à 380 °C,
   - que le catalyseur en vrac à lit fixe 1 soit constitué d'au moins deux zones de catalyseur en vrac à lit fixe se succédant spatialement, l'activité spécifique en volume à l'intérieur d'une zone de catalyseur en vrac à lit fixe étant sensiblement constante et augmentant brusquement dans le sens d'écoulement du mélange de gaz réactionnel 1 lors du passage d'une zone de catalyseur en vrac à lit fixe à une autre zone de catalyseur en vrac à lit fixe,
   - que la zone de température A s'étende jusqu'à une conversion du propène de 40 à 80 % en moles,
   - que, lors de l'unique passage du mélange initial de gaz réactionnel 1 à travers l'ensemble du catalyseur en vrac à lit fixe 1, la conversion de propène soit ≥ 90 % en mole et que la sélectivité de la formation d'acroléine ainsi que de la formation de produits secondaires d'acide acrylique représente, au total et par rapport au propène converti, une proportion ≥ 90 % en moles,
   - que la séquence chronologique selon laquelle le mélange de gaz réactionnel 1 traverse les zones de température A, B corresponde à la séquence alphabétique des zones de température A, B,
   - que la charge du catalyseur en vrac à lit fixe 1 avec le propène contenu dans le mélange initial de gaz réactionnel 1 représente une proportion ≥ 90 Nl de propène/l de catalyseur en vrac à lit fixe.h, et

- que la différence $T^{maxA}$ - $T^{maXB}$, formée de la température maximale $T^{maxA}$, que le mélange de gaz réactionnel 1 présente à l'intérieur de la zone de température A, et la température maximale $T^{maxB}$, que le mélange de gaz réactionnel 1 présenté à l'intérieur de la zone de température B, soit ≥ 0 °C,

on diminue la température du mélange de gaz produit quittant la première étape réactionnelle éventuellement par refroidissement et on ajoute au mélange de gaz produit éventuellement de l'oxygène moléculaire et/ou un gaz inerte, et ensuite on achemine le mélange initial de gaz réactionnel 2 contenant le mélange de gaz produit sous forme d'acroléine, d'oxygène moléculaire et d'au moins un gaz inerte, l'oxygène moléculaire et l'acroléine étant présents selon un rapport molaire $O_2:C_3H_4O \geq 0,5$, dans une deuxième étape réactionnelle sur un catalyseur en vrac à lit fixe 2, dont la masse active est au moins un oxyde de plusieurs métaux contenant les éléments Mo et V, de sorte :

- que le catalyseur en vrac à lit fixe 2 soit agencé dans deux zones de température C, D se succédant spatialement,
- que la température de la zone de température C ainsi que la température de la zone de température D se situent dans la plage de 230 à 320 °C,
- que le catalyseur en vrac à lit fixe 2 soit constitué d'au moins deux zones de catalyseur en vrac à lit fixe se succédant spatialement, l'activité spécifique en volume à l'intérieur d'une zone de catalyseur en vrac à lit fixe étant sensiblement constante et augmentant brusquement dans le sens d'écoulement du mélange de gaz réactionnel 2 lors du passage d'une zone de catalyseur en vrac à lit fixe à une autre zone de catalyseur en vrac à lit fixe,
- que la zone de température C s'étende jusqu'à une conversion de l'acroléine de 45 à 85 % en moles,
- que, lors de l'unique passage du mélange initial de gaz réactionnel 2 à travers l'ensemble du catalyseur en vrac à lit fixe 2, la conversion d'acroléine soit ≥ 90 % en moles et que la sélectivité de la formation d'acide acrylique, par rapport au propène converti dans les deux étapes réactionnelles, soit ≥ 80 % en moles,
- que la séquence chronologique, selon laquelle le mélange de gaz réactionnel 1 traverse les zones de température C, D, corresponde à la séquence alphabétique des zones de température C, D,
- que la charge du catalyseur en vrac à lit fixe 2 avec l'acroléine contenue dans le mélange initial de gaz réactionnel 2 représente 70 N1 d'acroléine/l de catalyseur en vrac à lit fixe 2.h, et
- que la différence $T^{maxC}$ - $T^{maxD}$, formée de la température maximale $T^{maxC}$, que le mélange de gaz réactionnel présente à l'intérieur de la zone de température C, et la température maximale $T^{maxD}$, que le mélange de gaz réactionnel présente à l'intérieur de la zone de température D, soit ≥ 0 °C,

**caractérisé en ce que** ni le passage de la zone de température A à la zone de température B dans le catalyseur en vrac à lit fixe 1, ni le passage de la zone de température C à la zone de température D dans le catalyseur en vrac à lit fixe 2 ne coïncide avec un passage d'une zone de catalyseur en vrac à lit fixe dans une autre zone de catalyseur en vrac à lit fixe.

2. Procédé selon la revendication 1, **caractérisé en ce que** $T^{maxA}$ - $T^{maxB}$ ≥ 3 °C et ≤ 70 °C.

3. Procédé selon la revendication 1 ou 2, **caractérisé en ce que** $T^{maxC}$ - $T^{maxD}$ ≥ 3 °C et ≤ 60 °C.

4. Procédé selon l'une quelconque des revendications 1 à 3, **caractérisé en ce que** la charge de propène dans le catalyseur en vrac à lit fixe 1 est ≥ 90 Nl/l·h et < 160 Nl/l·h.

5. Procédé selon l'une quelconque des revendications 1 à 3, **caractérisé en ce que** la charge de propène dans le catalyseur en vrac à lit fixe 1 est ≥ 160 Nl/l·h et ≤ 300 Nl/l·h.

6. Procédé selon l'une quelconque des revendications 1 à 5, **caractérisé en ce que** la composition chimique de la masse active utilisée ne change pas dans l'ensemble du catalyseur en vrac à lit fixe 1.

7. Procédé selon l'une quelconque des revendications 1 à 6, **caractérisé en ce que** l'ensemble du catalyseur en vrac à lit fixe 1 ne comprend pas plus de quatre zones de catalyseur en vrac à lit fixe.

8. Procédé selon l'une quelconque des revendications 1 à 7, **caractérisé en ce que**, pour une masse active homogène dans l'ensemble du catalyseur en vrac à lit fixe 1, la masse active spécifique en volume augmente d'au moins 5 % en poids lors du passage d'une zone de catalyseur en vrac à lit fixe à une autre zone de catalyseur en vrac à lit fixe du catalyseur en vrac à lit fixe 1 dans le sens d'écoulement du mélange de gaz réactionnel 1.

**9.** Procédé selon l'une quelconque des revendications 1 à 8, **caractérisé en ce que**, pour une masse active homogène dans l'ensemble du catalyseur en vrac à lit fixe 1, la différence de la masse active spécifique en volume entre la zone de catalyseur en vrac à lit fixe du catalyseur en vrac à lit fixe 1 ayant la plus faible activité spécifique en volume et la zone de catalyseur en vrac à lit fixe du catalyseur en vrac à lit fixe 1 ayant l'activité spécifique en volume la plus élevée ne dépasse pas 40 % en poids.

**10.** Procédé selon l'une quelconque des revendications 1 à 9, **caractérisé en ce que** la dernière zone de catalyseur en vrac à lit fixe du catalyseur en vrac à lit fixe 1 dans le sens d'écoulement du mélange de gaz réactionnel 1 n'est pas diluée et n'est constituée que de corps de moulage de catalyseur.

**11.** Procédé selon l'une quelconque des revendications 1 à 10, **caractérisé en ce que** la zone de catalyseur en vrac à lit fixe ayant l'activité spécifique en volume la plus élevée à l'intérieur du catalyseur en vrac à lit fixe 1 ne parvient pas dans la zone de température A.

**12.** Procédé selon l'une quelconque des revendications 1 à 11, **caractérisé en ce que**, dans le catalyseur en vrac à lit fixe 1, il n'y a aucun passage d'une zone de catalyseur en vrac à lit fixe à une autre zone de catalyseur en vrac à lit fixe dans la plage $X^1 \pm L^1 \cdot \dfrac{4}{100}$, $L^1$ représentant la longueur du catalyseur en vrac à lit fixe 1 et $X^1$ représentant l'endroit à l'intérieur du catalyseur en vrac à lit fixe 1, où a lieu le passage de la zone de température A à la zone de température B.

**13.** Procédé selon l'une quelconque des revendications 1 à 12, **caractérisé en ce que** la composition chimique de la masse active utilisée ne change pas dans l'ensemble du catalyseur en vrac à lit fixe 2.

**14.** Procédé selon l'une quelconque des revendications 1 à 13, **caractérisé en ce que** l'ensemble du catalyseur en vrac à lit fixe 2 ne comprend pas plus de quatre zones de catalyseur en vrac à lit fixe.

**15.** Procédé selon l'une quelconque des revendications 1 à 14, **caractérisé en ce que**, pour une masse active homogène sur l'ensemble du catalyseur en vrac à lit fixe 2, la masse active spécifique en volume augmente d'au moins 5 % en poids lors du passage d'une zone de catalyseur en vrac à lit fixe à une autre zone de catalyseur en vrac à lit fixe à l'intérieur du catalyseur en vrac à lit fixe 2 dans le sens d'écoulement du mélange de gaz réactionnel.

**16.** Procédé selon l'une quelconque des revendications 1 à 15, **caractérisé en ce que**, pour une masse active homogène sur l'ensemble du catalyseur en vrac à lit fixe 2, la différence de masse active spécifique en volume entre la zone de catalyseur en vrac à lit fixe à l'intérieur du catalyseur en vrac à lit fixe 2 ayant la plus faible activité spécifique en volume et la zone de catalyseur en vrac à lit fixe à l'intérieur du catalyseur en vrac à lit fixe 2 ayant l'activité spécifique en volume la plus élevée ne dépasse pas 40 % en poids.

**17.** Procédé selon l'une quelconque des revendications 1 à 16, **caractérisé en ce que** la dernière zone de catalyseur en vrac à lit fixe dans le sens d'écoulement du mélange de gaz réactionnel n'est pas diluée et n'est constituée que de corps de moulage de catalyseur.

**18.** Procédé selon l'une quelconque des revendications 1 à 17, **caractérisé en ce que** la zone de catalyseur en vrac à lit fixe ayant l'activité spécifique en volume la plus élevée ne parvient pas dans la zone de température C.

**19.** Procédé selon l'une quelconque des revendications 1 à 18, **caractérisé en ce que** dans le catalyseur en vrac à lit fixe 2 il n'y a aucun passage d'une zone de catalyseur en vrac à lit fixe à une autre zone de catalyseur en vrac à lit fixe dans la plage $X^2 \pm L^2 \dfrac{4}{100}$, $L^2$ représentant la longueur du catalyseur en vrac à lit fixe 2 et $X^2$ représentant l'endroit à l'intérieur du catalyseur en vrac à lit fixe, où a lieu le passage de la zone de température C à la zone de température D.

**EP 1 611 077 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19927624 A
- DE 19948523 A
- WO 0053557 A
- DE 19948248 A
- WO 0053558 A
- DE 3042468 A
- EP 1106598 A
- DE 19955176 A
- DE 10101695 A
- DE 19955168 A
- EP 700714 A
- DE 10046957 A
- DE 10063162 A
- DE 3338380 C
- DE 19902562 A
- EP 15565 A
- DE 2380765 C
- EP 807465 A
- EP 279374 A
- DE 3300044 A
- EP 575897 A
- US 4438217 A
- DE 19855913 A
- WO 9824746 A
- DE 19746210 A
- JP 3294239 A
- EP 293224 A
- WO 02062737 A
- DE 4023239 A
- DE 2909671 A

- EP 293859 A
- EP 714700 A
- DE 10046928 A
- DE 19815281 A
- DE 4335973 A
- EP 668104 A
- DE 19736105 A
- DE 19740493 A
- DE 19528646 A
- DE 4442346 A
- DE 2830765 C
- DE 2513405 C
- US 3147084 A
- DE 2201528 A
- EP 383224 A
- DE 2903218 A
- EP 700893 A
- EP 468290 B
- DE 2201528 B
- EP 382098 A
- WO 0136364 A
- DE 10302715 A
- DE 10261186 A
- EP 0214187 W
- EP 0214188 W
- EP 214189 W
- EP 873783 A
- EP 1270065 A